(19) Europäisches Patentamt
European Patent Office
Office européen des brevets

(11) Numéro de publication: **0 237 397**
**B1**

# (12) FASCICULE DE BREVET EUROPEEN

(45) Date de publication du fascicule du brevet: **07.02.90**

(21) Numéro de dépôt: **87400406.2**

(22) Date de dépôt: **24.02.87**

(51) Int. Cl.[4]: **C07J 1/00**, C07J 21/00, A61K 31/565, A61K 31/585, C07J 31/00, C07J 33/00, C07J 41/00, C07J 43/00, C07J 51/00, C07J 53/00

(54) **Nouveaux stéroïdes 9(11) saturés substitués en position 10 par un radical comportant une triple liaison, leur procédé de préparation, leur application comme médicaments, les compositions pharmaceutiques les renfermant.**

(30) Priorité: **24.02.86 FR 8602510**

(43) Date de publication de la demande: **16.09.87 Bulletin 87/38**

(45) Mention de la délivrance du brevet: **07.02.90 Bulletin 90/6**

(84) Etats contractants désignés: **CH DE FR GB IT LI NL**

(56) Documents cités:
**EP-A- 0 176 399**
**GB-A- 2 078 749**
**US-A- 3 218 316**

**STEROIDS, vol. 39, no. 3, mars 1982, pages 325-344, San Francisco, US; P.A. MARCOTTE et al.: "Synthesis and evaluation of 10beta-substituted 4-estrene-3,17-diones as inhibitors of human placental microsomal aromatase"**

(73) Titulaire: **ROUSSEL-UCLAF, 35, boulevard des Invalides, F-75007 Paris(FR)**

(72) Inventeur: **Nedelec, Lucien, 45, Boulevard de L'Ouest, F-93340 Le Raincy(FR)**
Inventeur: **Nique, Francois, 7, Allée Pierre Brossolette, F-93320 Pavillons-Sous-Bois(FR)**
Inventeur: **Moura, Anne-Marie, 15-29, rue Guilleminot, F-75014 Paris(FR)**

(74) Mandataire: **Bourgouin, André et al, Département des Brevets ROUSSEL UCLAF B.P. no 9, F-93230 Romainville(FR)**

**EP 0 237 397 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne de nouveaux stéroides 9 (11) saturés substitués en position 10 par un radical comportant une triple liaison, leur procédé de préparation, leur application comme médicament et les compositions pharmaceutiques les renfermant.

Dans le brevet américain USP 3 218 316 sont décrits des produits stéroïdes comportant en position 10 un radical éthynyle. Ces produits qui sont différents des produits décrits ci-après dans la présente demande ne sont pas présentés comme possèdant les mêmes propriétés pharmacologiques que les produits décrits ci-après.

L'invention a pour objet les produits de formule générale I:

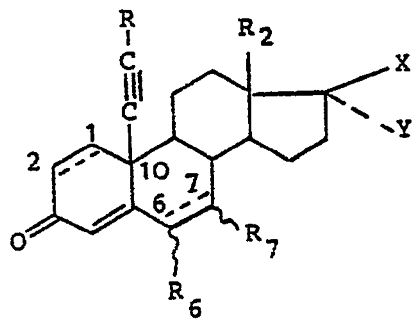

dans laquelle R représente:
– soit un atome d'hydrogène,
– soit un radical alkyle, alkényle ou alkynyle ayant au plus 8 atomes de carbone, éventuellement substitué par un radical choisi parmi les radicaux hydroxyle, carboxy, méthoxycarbonyle, éthoxycarbonyle, amino, tritylamino, chloroacétylamino, trifluoroacétylamino, trichloroéthoxycarbonylamino, méthylamino, diméthylamino ou halogène,
– soit un radical aryle ou aralkyle éventuellement substitué par un radical choisi parmi les radicaux hydroxyle, carboxy, méthoxycarbonyle, éthoxycarbonyle, amino, méthylamino, diméthylamino, méthyle méthoxy ou méthylthio,
– soit un radical alkoxy, carboxy, méthoxycarbonyle, éthoxycarbonyle ou dialkylamino,
– soit un atome d'halogène,
– soit un radical trialkylsilyle,
$R_6$ et $R_7$ sont tels que, soit $R_6$ et $R_7$ forment avec les carbones qui les portent un radical cyclopropyle, soit $R_6$ représente un atome d'hydrogène et $R_7$ représente le radical $R_1$, $R_1$ représentant:
– soit un atome d'hydrogène,
– soit un radical alkyle, alkényle ou alkynyle ayant au plus 6 atomes de carbone, éventuellement substitué par un radical choisi parmi les radicaux hydroxyle, carboxy, méthoxycarbonyle, éthoxycarbonyle, amino, tritylamino, chloroacétylamino, trifluoroacétylamino, trichloroéthoxycarbonylamino, méthylamino, diméthylamino ou halogène,
– soit un radical acétylthio,
$R_2$ représente un radical méthyle ou éthyle,
X et Y sont tels que:
– soit X représente un radical hydroxyle, acétyloxy, propionyloxy, méthoxy ou éthoxy et Y représente ou bien un radical $-CH_2CH_2CO_2M$ dans lequel M représente un atome d'hydrogène, un atome de métal alcalin ou un radical ammonium,
ou bien un radical $-CH_2CH_2-CH_2OH$
– soit X et Y représentent ensemble un radical:

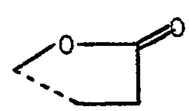 ou 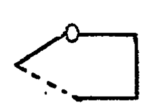

– soit X et Y représentent ensemble un radical

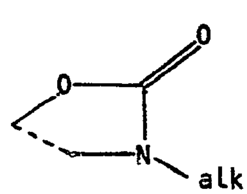

dans lequel alk représente un radical alkyle renfermant au plus 8 atomes de carbone,
– soit X et Y représentent ensemble un radical

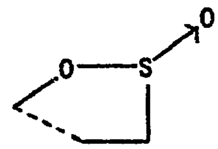

ou X représente un radical hydroxyle et Y un radical

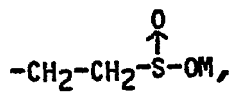

M étant défini comme ci-dessus,
– soit X représente un radical hydroxyle, acétyloxy, propionyloxy, méthoxy ou éthoxy et Y représente, ou bien un atome d'hydrogène ou bien Y représente $R_4$, $R_4$ représentant un radical alkyle, alkényle ou alkynyle ayant au plus 6 atomes de carbone,
les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle,
les traits ondulés en position 6 et 7 indiquent que les substituants $R_6$ et $R_7$ peuvent se trouver dans l'une des positions possibles alpha ou béta, étant entendu que R ne peut pas représenter un atome d'hydrogène lorsque $R_6$ et $R_7$ représentent chacun un atome d'hydrogène, $R_2$ représente un radical méthyle, X représente un radical hydroxy, acétyloxy, propionyloxy, méthoxy ou éthoxy et Y représente un atome d'hydrogène ou $R_4$ et les traits pointillés en position 1 (2) et 6 (7) ne représentent pas ensemble une seconde liaison entre les carbones qui les portent.

L'invention a pour objet, parmi les produits de formule I, ceux répondant à la formule $I_1$:

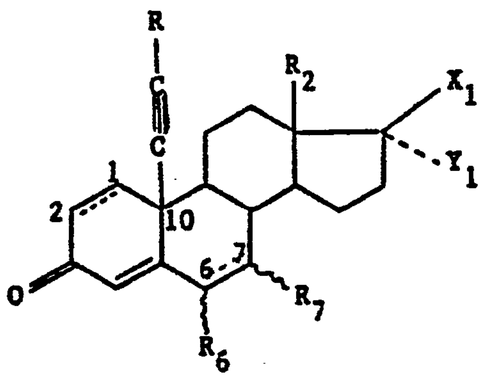
($I_1$)

dans laquelle R, $R_6$, $R_7$, $R_2$, les traits pointillés en 1 (2) et 6 (7) et les traits ondulés en 6 et 7 ont les significations indiquées précédemment,
$X_1$ et $Y_1$ sont tels que:
– soit $X_1$ représente un radical hydroxyle, acetyloxy, propionyloxy, méthoxy ou éthoxy, et $Y_1$ représente ou bien un radical $-CH_2CH_2CO_2M$ dans lequel M représente un atome d'hydrogène, un atome de métal alcalin ou un radical ammonium, ou bien un radical $-CH_2CH_2-CH_2OH$,
– soit $X_1$ et $Y_1$ représentent ensemble un radical:

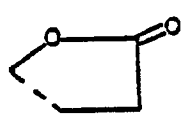 ou 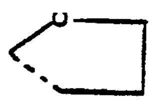

– soit $X_1$ représente un radical hydroxyle, acétyloxy, propionyloxy, méthoxy ou éthoxy et $Y_1$ représente, ou bien un atome d'hydrogène ou bien $Y_1$ représente $R_4$, $R_4$ représentant un radical alkyle, alkényle ou alkynyle ayant au plus 6 atomes de carbone,
étant entendu que R ne peut pas représenter un atome d'hydrogène lorsque $R_6$ et $R_7$ représentent chacun un atome d'hydrogène, $R_2$ représente un radical méthyle, $X_1$ représente un radical hydroxy, acétyloxy, propionyloxy, méthoxy ou éthoxy, $Y_1$ représente un atome d'hydrogène ou $R_4$ et les traits pointillés en position 1 (2) et 6 (7) ne représentent pas une seconde liaison entre les carbones qui les portent.

Parmi les valeurs de R on peut citer les radicaux méthyle, éthyle, propyle, isopropyle, butyle, butyle secondaire, tert-butyle et les radicaux pentyle, hexyle, heptyle ou octyle éventuellement ramifiés.

On peut également citer les radicaux alkényles suivants: vinyle, allyle, 1-propényle, butényle, les radicaux alkynyles tels que éthynyle ou propargyle ou butynyle.

Ces differents radicaux peuvent être substitués par des atomes d'halogènes, de préférence chlore ou brome.

Les radicaux aryles et aralkyles que peut également représenter R sont de préférence un radical phényle, benzyle ou phényléthyle. Ces radicaux peuvent également être substitués, par exemple par les radicaux hydroxyle, carboxy éventuellement estérifiés, amino, mono ou dialkylamino, alkyle tel que méthyle, alkoxy tel que méthoxy, alkylthio tel que méthylthio.

La valeur alkoxy que peut représenter R peut-être par exemple un radical méthoxy, éthoxy, propyloxy ou tert-butyloxy.

La valeur trialkylsilyle préférée est la valeur triméthylsilyle.

Les valeurs que peut représenter le radical $R_1$ peuvent également être choisies dans les valeurs citées précédemment pour R. Il en est de même pour $R_4$.

M représente de préférence un atome de sodium, de potassium ou de lithium.

Lorsque $R_1$ représente un substituant différent d'un atome d'hydrogène le substituant est de préférence en position 7. Le radical cyclopropyle que $R_6$ et $R_7$ peuvent former avec les carbones qui les portent est de préférence 6 béta, 7 béta.

Les composés de formule I dans laquelle X et Y représentent ensemble un radical

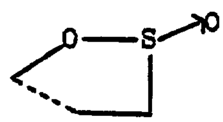

existent sous forme de deux diastéréoisomères au niveau de l'atome de soufre, éventuellement séparables l'un de l'autre et dénommés par convention isomères A et B, l'isomère A étant l'isomère dont le point de fusion est le plus élevé.

Parmi les produits de formule I, on préfère d'abord les produits de formule:

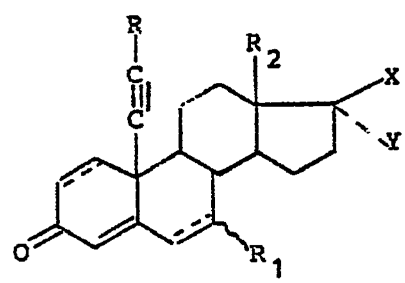

dans laquelle R, $R_1$, $R_2$, X, Y, les traits pointillés et les traits ondulés ont la signification indiquée ci-dessus.

L'invention a plus particulièrement pour objet les produits de formule I telle que décrite ci-dessus dans laquelle R est choisi dans le groupe formé par l'atome d'hydrogène, les radicaux alkyles ayant de 1 à 3 atomes de carbone, le radical hydroxyméthyle et le radical phényle ainsi que les produits de formule I dans laquelle le substituant $R_1$ est choisi dans le groupe formé par l'atome d'hydrogène, les radicaux alkyles ayant de 1 à 4 atomes de carbone et le radical acétylthio et X et Y sont tels que soit X représente un radical hydroxy et Y représente ou bien un radical $CH_2CH_2CO_2M'$ dans lequel M' représente un atome d'hydrogène ou atome de métal alcalin, ou bien Y représente un atome d'hydrogène,

– soit X et Y représentent un radical:

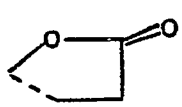

Plus particulièrement, on préfère les produits dans lesquels R représente un atome d'hydrogène ou un radical méthyle, $R_2$ représente un radical méthyle et X et Y sont tels que soit X représente un radical OH et Y représente un atome d'hydrogène lorsque R est différent d'un atome d'hydrogène, soit X et Y représentent ensemble un radical:

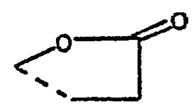

L'invention a plus particulièrement pour objet les produits décrits ci-après dans les exemples, à savoir:

– la gamma-lactone de l'acide 10 béta-éthynyl 17 béta-hydroxy 3-oxo 19-nor 17 alpha-pregn 4-èn 21-carboxylique,

– la 17 béta-hydroxy 10 béta-(1-propynyl) estr-4-èn 3-one,

– la gamma-lactone de l'acide 17 béta-hydroxy 3-oxo 10 béta-(1-propynyl) 19-nor 17 alpha-pregn 4-èn 21-carboxylique.

L'invention a également pour objet, un procédé de préparation des produits de formule I, telle que définie ci-dessus étant entendu que R ne peut pas représenter un atome d'hydrogène, lorsque $R_6$ et $R_7$ représentent chacun un atome d'hydrogène, $R_2$ représente un radical méthyle, X représente un radical hydroxy, acétyloxy, propionyloxy, méthoxy ou éthoxy, Y représente un atome d'hydrogène ou $R_4$ et les traits pointillés en position 1 (2) et 6 (7) ne représentent pas ensemble une seconde liaison entre les carbones qui les portent, caractérisé en ce que l'on traite un produit de formule II:

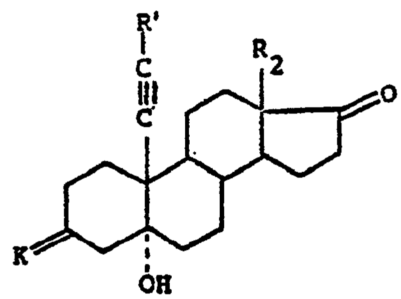

(II)

dans laquelle R′ représente ou bien R, R ayant la signification indiquée ci-dessus, ou bien R dans lequel les fonctions réactives sont protégées, $R_2$ a la signification indiquée ci-dessus et K représente un groupement protecteur de la fonction cétone,

soit d'abord par un halogénure de triméthylsulfonium en présence d'une base forte, puis par un dérivé métallique de l'acétonitrile et enfin par une base, puis par un acide,

soit d'abord par un réactif de formule:

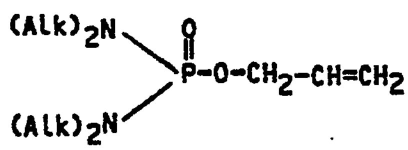

dans lequel Alk représente un radical alkyle ayant de 1 à 4 atomes de carbone en présence d'une base forte puis par un acide, pour obtenir un produit de formule $I_A$:

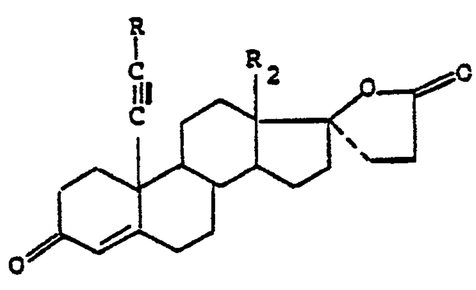

($I_A$)

soit par un réactif de formule:

$XMg\text{-}CH_2\text{-}CH_2\text{-}CH_2\text{-}OB$

dans lequel X représente un atome d'halogène et B représente un groupement protecteur du radical hydroxyle ou un alcoolate de magnésium pour obtenir, après traitement par un acide, un produit de formule $I'_A$:

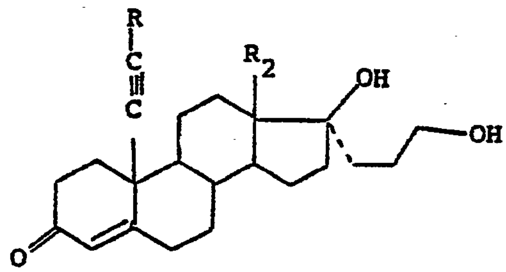

$(I'_A)$

produit de formule $I'_A$ que l'on traite éventuellement par un halogénure d'acide sulfonique en présence d'une base pour obtenir un produit de formule $I''_A$ :

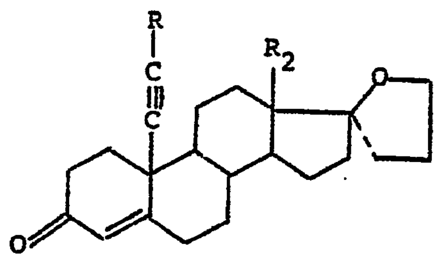

$(I''_A)$

soit par un réactif de réduction, puis par un acide, pour obtenir un produit de formule $I_{3A}$ :

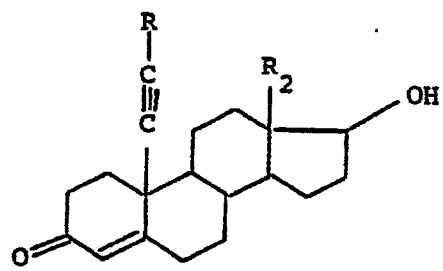

$(I_{3A})$

soit par un organométallique $R_4MgX$, X représentant un atome d'halogène, puis par un acide, pour obtenir un produit de formule $I_{4A}$ :

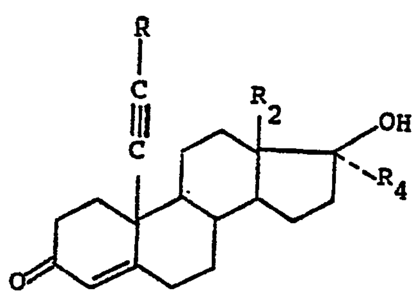

$(I_{4A})$

soit d'abord par un halogénure de triméthylsulfonium en présence d'une base forte, puis par une amine primaire de formule $H_2N$-alk, alk étant défini comme précédemment, en présence d'un acide, puis par un chloroformiate d'alkyle, par une base forte et enfin par un acide, pour obtenir un produit de formule $I_{5A}$ :

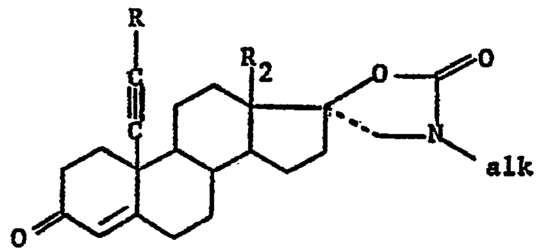

$(I_{5A})$

soit d'abord par un halogénure de triméthylsulfonium en présence d'une base forte, puis par le méthyl terbutyl sulfoxyde en présence de n-butyl-lithium pour obtenir un produit de formule :

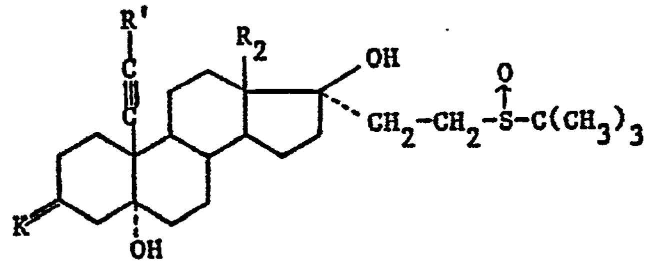

sous forme d'un mélange de deux diastéréoisomères au niveau de l'atome de soufre, sépare si désiré les deux diastéréoisomères, puis soumet soit leur mélange, soit chacun d'eux séparément, à l'action d'un acide, pour obtenir un produit de formule :

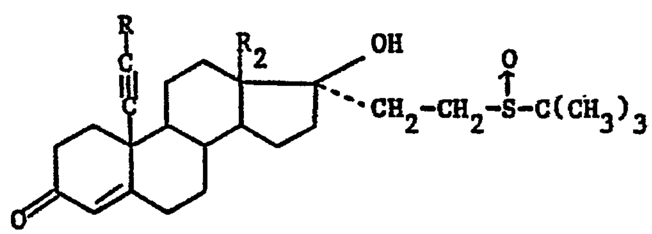

sous forme d'un mélange de deux diastéréoisomères ou d'un diastéréoisomère, sépare le cas échéant, les diastéréoisomères, puis soumet soit leur mélange, soit chacun d'eux séparément, à l'action du N-chloro ou du N-bromo succinimide, pour obtenir un produit de formule $I_{6A}$ :

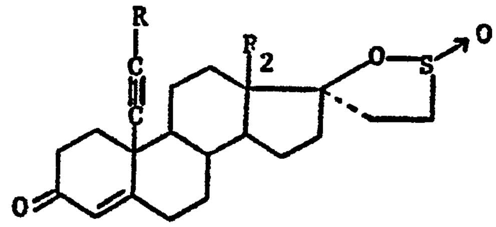

$(I_{6A})$

et que, si désiré, l'on traite les produits de formules $I_A$, $I'_A$, $I''_A$, $I_{3A}$, $I_{4A}$, $I_{5A}$ et $I_{6A}$ par un orthoformiate d'alkyle en présence d'un acide, puis par un agent de déhydrogénation, pour obtenir les produits de formules $I_B$, $I'_B$, $I''_B$, $I_{3B}$, $I_{4B}$, $I_{5B}$ et $I_{6B}$ suivantes:

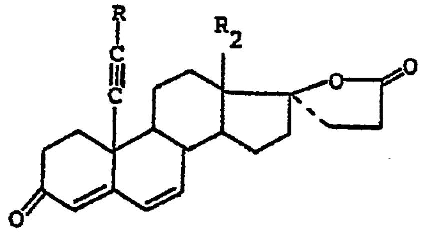

$I_B$

;

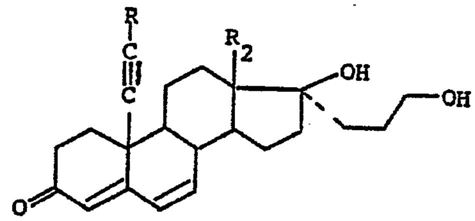

$I'_B$

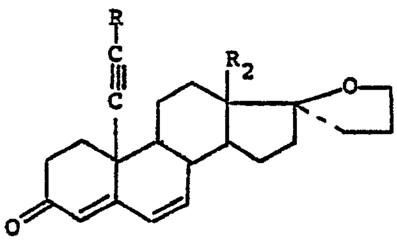

$I''_B$

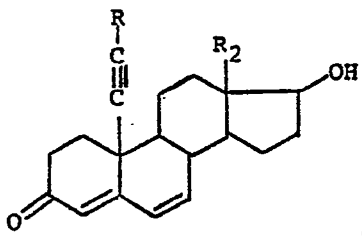

$I_{3B}$

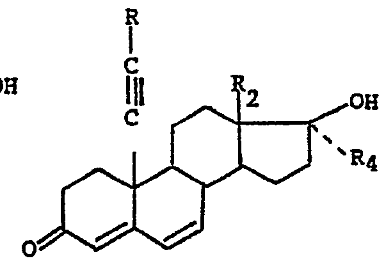

$I_{4B}$

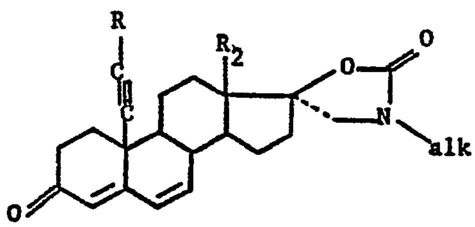

$I_{5B}$

.

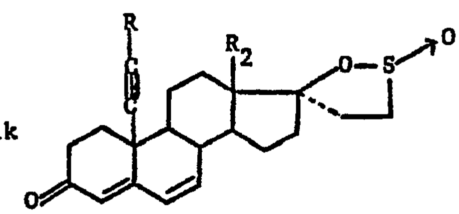

$I_{6B}$

produits de formules $I_B$, $I'_B$, $I''_B$, $I_{3B}$, $I_{4B}$, $I_{5B}$ et $I_{6B}$ que l'on traite si désiré :

- <u>ou bien</u> par un organo magnésien de formule $R_1MgX'$ éventuellement en présence d'un sel de cuivre, formule dans laquelle $R_1$ à la signification indiquée ci-dessus et X' représente un atome d'halogène,
- <u>ou bien</u> par un organo métallique de formule $(R_1)_2$ CuLi puis par un acide,

pour obtenir les produits de formules $I_C$, $I'_C$, $I''_C$, $I_{3C}$ $I_{4C}$, $I_{5C}$ et $I_{6C}$ suivantes :

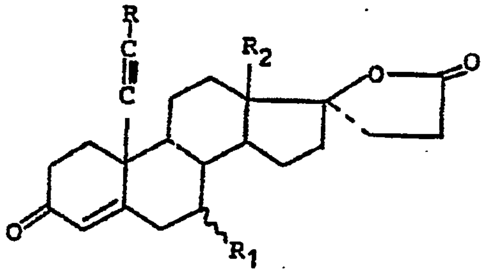

$I_C$

;

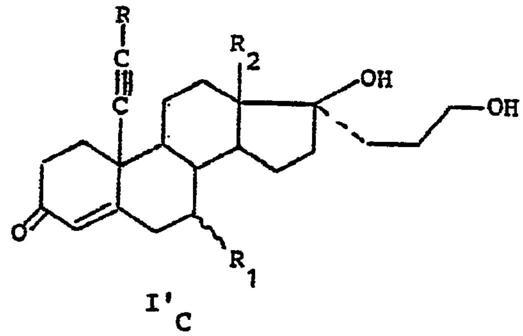

$I'_C$

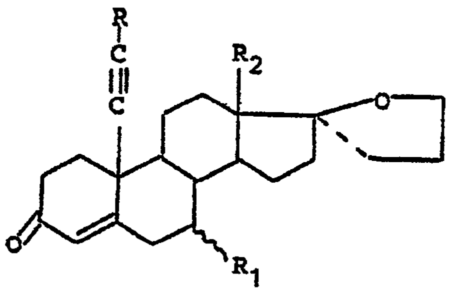

$I''_C$

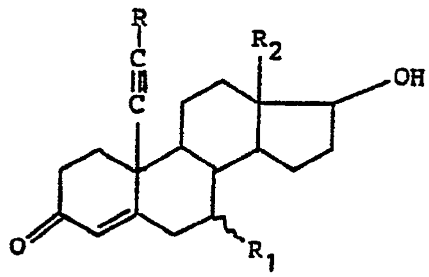

$I_{3C}$

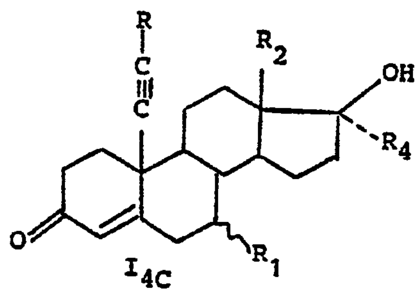

$I_{4C}$

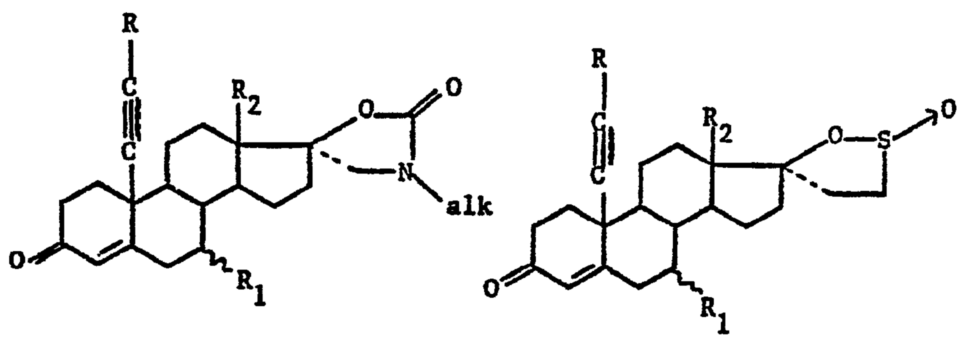

$I_{5C}$ $I_{6C}$

sous forme d'un mélange 7 alpha et 7 béta, mélange que, si désiré, l'on sépare et que, si désiré, l'on soumet les produits 7 béta à un réactif de déshydrogénation pour obtenir les produits delta 6(7) correspondants :

- ou bien l'on soumet les produits de formules $I_B$, $I'_B$, $I''_B$, $I_{3B}$, $I_{4B}$, $I_{5B}$ et $I_{6B}$ à l'action d'un réactif choisi dans le groupe constitué par l'iodure de triméthylsulfonium et l'iodure de triméthylsulfoxonium en présence d'une base forte pour obtenir les produits de formules $I_D$, $I'_D$, $I''_D$, $I_{3D}$, $I_{4D}$, $I_{5D}$ et $I_{6D}$ suivantes :

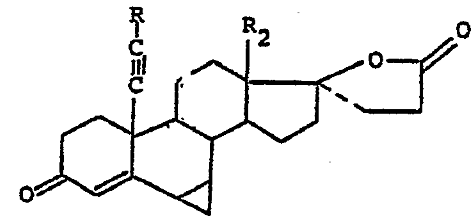

$I_D$

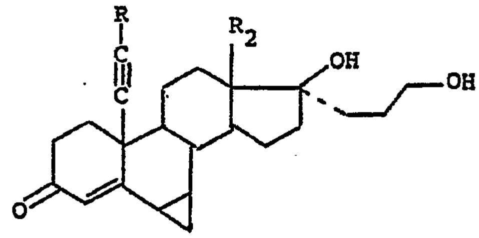

$I'_D$

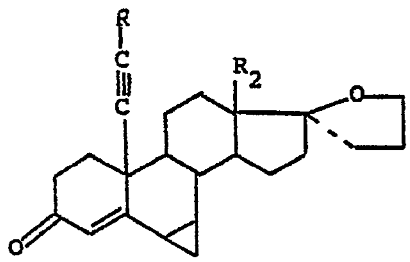

$I''_D$

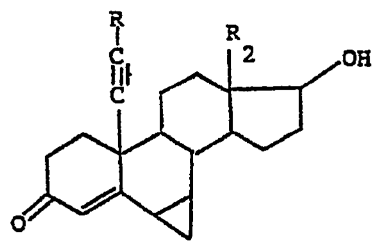

$I_{3D}$

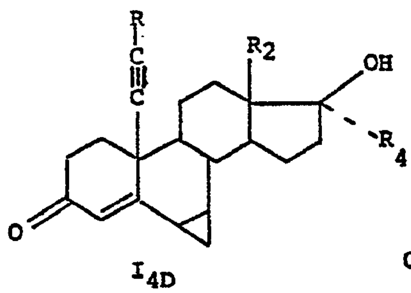

$I_{4D}$

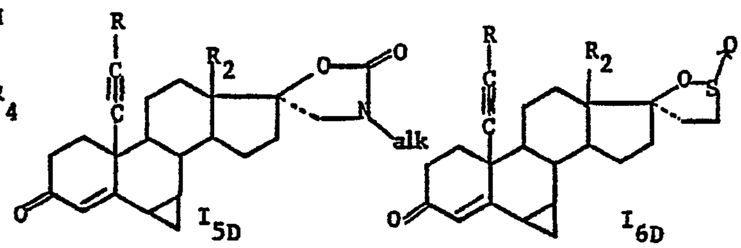

$I_{5D}$ $I_{6D}$

sous forme d'un mélange 6 alpha, 7 alpha et 6 béta, 7 béta et sépare, si désiré, les isomères ainsi obtenus et que si désiré l'on traite les produits $I_A$ $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$, par un réactif de déshydrogénation ou par un microorganisme susceptible de déshydrogéner la molécule en position 1 (2) pour obtenir les produits correspondants comportant une insaturation en position 1(2) et que, si désiré, l'on traite les produits de formules $I_A$, $I_B$, $I_C$ et $I_D$ et les produits correspondants comportant une insaturation en position 1(2) à l'aide d'un hydroxyde alcalin ou de l'ammoniaque pour obtenir les produits correspondants dans lesquels X représente un radical hydroxyle et Y représente un radical $-CH_2CH_2CO_2M'$ dans lequel M' représente un atome de métal alcalin ou un radical ammonium et, si désiré, soumet les produits ainsi obtenus à l'action d'un agent acide pour obtenir les produits dans lesquels X représente un radical hydroxyle et Y représente un radical $-CH_2CH_2CO_2H$, et que si désiré l'on traite les produits dans lesquels le substituant en position 10 comporte un substituant R du type hydroxyalkyle, par un tétra-bromure ou tétrachlorure de carbone, en présence de triphénylphosphine, pour obtenir les dérivés bromés et chlorés correspondants, et que si désiré, l'on traite les produits comportant en position 10 un substituant éthynyl par un halo-succinimide pour obtenir les produits correspondants comportant en position 10 un substituant -C=C-Hal, et que si désiré l'on acyle ou éthérifie le radical hydroxyle en position 17 béta des produits comportant en position 17 alpha un atome d'hydrogène, un radical $R_4$ ou un radical $-CH_2-CH_2-CH_2OH$.

Les groupements protecteurs des fonctions que peut comporter R, par exemple les groupements hydroxy ou amino, sont choisis parmi les groupements connus.

Le groupement protecteur de la fonction cétone que représente K peutêtre un cétal, un thiocétal, un oxime ou un alkoxime. On utilise de préférence un cétal tel que le bis méthoxy ou un cétal cyclique tel que l'éthylène dioxy. Ces groupements sont éliminés en milieu acide.

L'halogénure de triméthylsulfonium que l'on utilise de préférence estl'iodure. La base forte en présence de laquelle on agit est de préférence le terbutylate de potassium.

On fait ensuite agir un dérivé métallique de l'acétonitrile qui peut par exemple être le dérivé lithien préparé en présence de butyllithium. Le dérivé ainsi obtenu qui comporte en position 17 un radical cyanoéthyle est ensuite soumis à l'action d'une base qui peut être par exemple la soude ou la potasse et on acidifie le produit obtenu par l'acide chlorhydrique de préférence.

L'action du tétralkyl phosphorodiamidate d'allyle, de préférence le tétraméthyle, sur le produit de formule II est effectuée selon la méthode décrite par STURTZ et YAOUANC, Synthesis 1980 p. 289. La base forte en présence de laquelle on agit est de préférence le butyllithium.

On peut également opérer en plus en présence de diazabicyclo octane (DABCO) ou d'un éther couronne. On acidifie ensuite à l'aide d'acide chlorhydrique, de préférence.

L'action du produit de formule XMg-$CH_2CH_2CH_2OB$ sur le produit de formule II est effectuée selon les méthodes classiques de préparation de magnésien. Le radical B peut représenter un groupement protecteur habituel du radical hydroxyle, tel que le tétrahydro-pyrannyle ou le terbutyle. B peut également représenter un sel de magnésium tel que MgCl. La préparation et l'utilisation d'un tel groupement sont décrites par CAHIEZ, ALEXAKIS, et NORMANT Tetr. lett. n° 33, 1978 p. 3013. On acidifie ensuite à l'aide d'un acide, l'acide chlorhydrique depréférence.

La transformation des produits de formule $I'_A$ en produits $I''_A$ est effectuée de préférence en présence de chlorure de tosyle et d'une base aminée telle que la pyridine.

La réduction des produits de formule II en produits de formule $I_{3A}$ est effectuée par les méthodes classiques. On peut utiliser par exemple un hydrure tel que le borohydrure de sodium.

L'hydrolyse du groupement protecteur K est effectuée par les méthodes classiques en milieu acide par exemple, par addition d'acide chlorhydrique.

L'addition du magnésien $R_4XMg$ est effectuée selon les méthodes classiques, on opère par exemple dans un solvant anhydre tel que le tétrahydrofuranne ou l'éther éthylique et à une température inférieure à la température ambiante. Comme pour les autres réactions indiquées précédemment on fait agir ensuite un acide tel que l'acide chlorhydrique pour régénérer la fonction cétone en position 3.

La base forte utilisée dans la cyclisation conduisant au produit $I_{5A}$ est de préférence la potasse méthanolique.

L'acide en présence duquel est effectuée la réaction avec l'amine est de préférence l'acide paratoluène sulfonique.

L'halogénure de triméthylsulfonium est de préférence un iodure et la base forte utilisée est la soude, la potasse ou le terbutylate de potassium.

La séparation des diastéréoisomères est effectuée par les moyens classiques de chromatographie ou de cristallisation.

L'action d'un orthoformiate sur les produits $I_A$, $I'_A$, $I''_A$, $I_{3A}$, $I_{4A}$, $I_{5A}$ et $I_{6A}$ a pour but de préparer les produits 3-alkoxy delta 3,5 de formules :

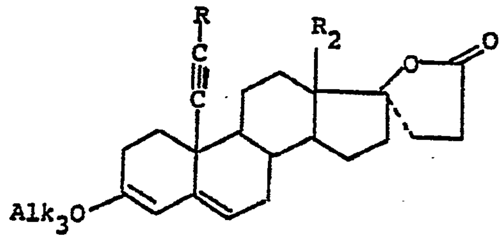

$I_{OA}$

;

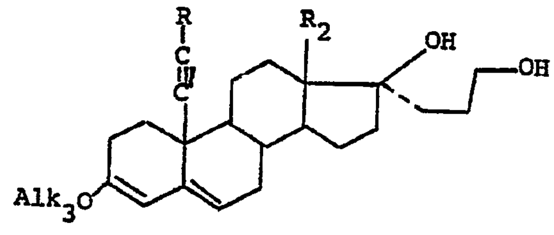

$I'_{OA}$

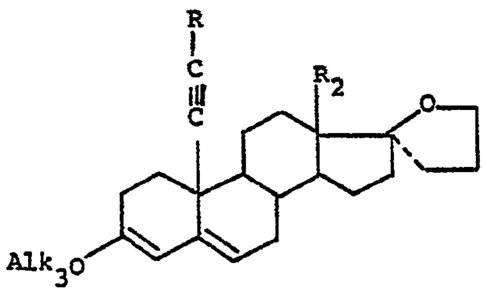

$I''_{OA}$

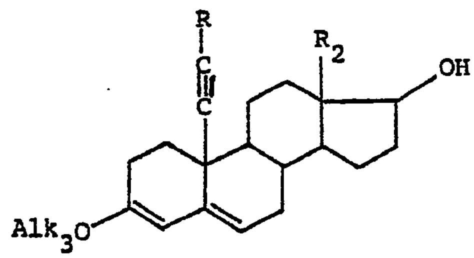

$I_{O3A}$

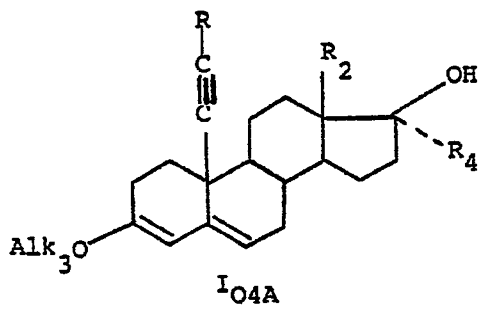

$I_{O4A}$

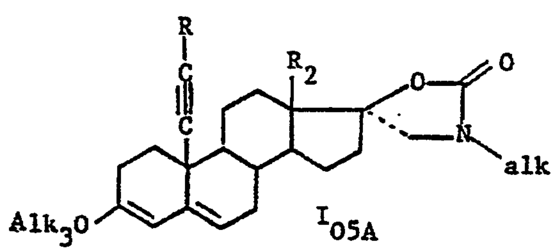

$I_{O5A}$

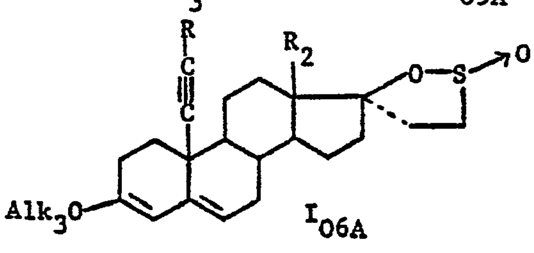

$I_{O6A}$

Dans les formules $I_{0A}$, $I'_{0A}$, $I''_{0A}$, $I_{03A}$, $I_{04A}$, $I_{05A}$ et $I_{06}$ les radicaux R, $R_2$ et $R_4$ ont la signification précédente, $Alk_3$ représente un radical alkyle ayant de 1 à 4 atomes de carbone. L'orthoformiate utilisé est de préférence l'orthoformiate d'éthyle en présence d'acide paratoluènesulfonique. L'agent de déshydrogénation que l'on utilise est de préférence le chloranile (tétrachloro 1,4-benzoquinone). On peut également utiliser le DDQ (2,3-dichloro 5,6-dicyano 1,4-benzoquinone). L'action du produit de formule $R_2Mgx'$ dans laquelle X' représente un atome de chlore, brome ou iode est effectuée de préférence en présence d'un sel cuivrique tel que l'acétate, le chlorure, le bromure cuivrique ou un sel cuivreux tel que le chlorure, le bromure ou l'iodure cuivreux.

L'acide que l'on utilise après action d'un produit de formule $(R_1)_2CuLi$ est l'acide chlorhydrique, nitrique ou sulfurique.

La séparation éventuelle des différents isomères est réalisée par chromatographie ou par cristallisation fractionnée. La déshydrogénation éventuelle des produits 7 béta est effectuée dans les conditions énoncées précédemment.

La base utilisée pour former l'ylure correspondant à l'iodure de triméthylsulfonium et l'iodure de triméthylsulfoxonium est l'hydrure de sodium ou le tert-butylate de potassium.

La séparation éventuelle est effectuée selon les méthodes classiques énoncées ci-dessus.

La transformation des produits $I_A$ à $I_{6A}$, $I_B$ à $I_{6B}$, $I_C$ à $I_{6C}$ et $I_D$ à $I_{6D}$ en produits comportant une insaturation en position 1 (2) est effectuée de préférence en faisant agir par voie biochimique, la bactérie "Arthrobacter simplex". On peut également utiliser un autre microorganisme. Dans ce cas, la réaction est effectuée de préférence en milieu aqueux tamponné.

On peut également utiliser la voie chimique en soumettant les produits à l'action d'un dérivé de la p-benzoquinone ou du chloranile, la réaction a lieu au sein, par exemple, d'une solution dans un solvant organique tel que le dioxanne, l'acétone, le benzène ou l'alcool tertbutylique.

On peut également utiliser, comme réactif de déshydrogénation, l'anhydride sélénieux ou phényle séléninique.

L'hydroxyde alcalin auquel on soumet éventuellement les produits de formules $I_A$, $I_B$ et $I_C$ ainsi que les produits correspondants comportant une insaturation en position 1 (2) est de préférence la soude ou la potasse.

L'agent acide auquel on soumet éventuellement les produits ainsi obtenus est l'acide chlorhydrique, sulfurique, nitrique ou acétique.

L'halosuccinimide que l'on fait agir avec les produits comportant en position 10 un substituant éthynyl est le N-bromo ou le N-chloro succinimide. On opère de préférence en présence de nitrate d'argent.

L'acylation éventuelle des produits comportant en position 17β un radical hydroxyle et en position 17 alpha un atome d'hydrogène ou un radical $R_4$ est effectuée selon les méthodes usuelles. On peut utiliser par exemple un anhydride ou un halogénure d'acyle tel que l'anhydride acétique ou le chlorure d'acétyle. L'éthérification des mêmes produits est effectuée également dans les conditions usuelles par exemple à l'aide d'un halogénure d'alkyle.

L'invention a également pour objet un procédé de préparation des produits de formule $I_{3A}$ telle que définie ci-dessus caractérisé en ce que l'on traite un produit de formule II' :

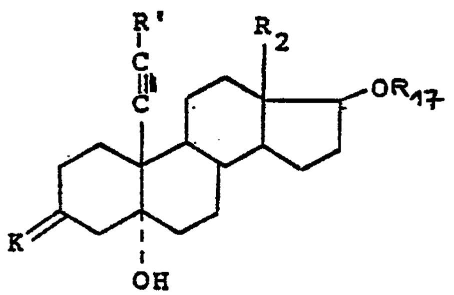

(II')

dans laquelle K, R' et $R_2$ ont la signification indiquée ci-dessus et $R_{17}$ représente un atome d'hydrogène ou un groupement protecteur du radical hydroxyle, par un acide et éventuellement par un réactif de déprotection du radical hydroxyle.

Lorsque $R_{17}$ représente un groupement protecteur du radical hydroxyle, celui-ci est choisi parmi les groupements connus. On préfère les groupes benzoyle et tétrahydropyrannyle. Les groupements sont normalement éliminés par le traitement acide visant à obtenir la fonction 3 cétone delta 4. Si ce n'est pas le cas, on traite les molécules de formule II' par un réactif de déprotection classique.

Comme indiqué ci-dessus le traitement acide préféré est l'addition d'acide chlorhydrique.

Les produits de formule II et II' utilisés au départ du procédé de la présente demande peuvent être préparés en faisant agir un lithien de formule R'-C≡C-Li sur un produit de formule III :

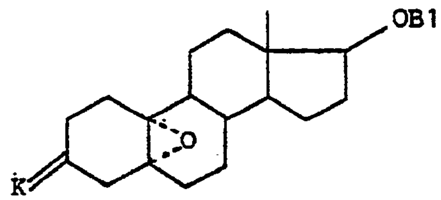

(III)

dans lequel $B_1$ représente un groupement protecteur du radical hydroxyle pour obtenir un produit de formule II' :

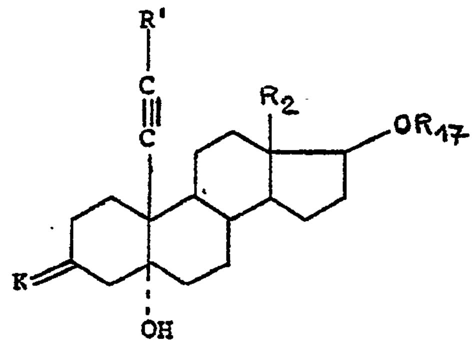

(II')

dans laquelle $R_{17}$ représente un atome d'hydrogène ou le radical $B_1$ précédent, produit que l'on soumet d'abord à une réaction de déprotection du radical hydroxyle si le groupement protecteur n'a pas été clivé lors de la réaction précédente, puis à une réaction d'oxydation, pour obtenir les produits de formule II.

Le groupement portecteur que peut représenter $B_1$ est de préférence un groupement acyle tel que benzoyle. Le lithien R'-C≡C-Li peut être préparé par les méthodes usuelles ; l'hydrolyse du groupement $B_1$ est également réalisée par les méthodes usuelles si le clivage n'est pas déjà intervenu lors de la réaction précédente. On peut utiliser une hydrolyse en milieu basique. L'oxydation en postition 17 est effectuée par exemple par utilisation de chlorochromate de pyridinium préparé selon Tet. Letters. N° 31(1975) p.2647. Un tel exemple de préparation est décrit dans le brevet européen 0.023.856 (ex. 5).

Les produits de formule III ainsi que certains produits de formule II' sont connus dans la littérature ou peuvent être préparés à partir de produits connus par des méthodes par analogie. On peut citer comme source pour la préparation de formule III, le brevet français BF 1.550.974 et pour la préparation de produits II' le brevet européen EP0.023.856 précité.

Les produits de formule générale I présentent de très intéressantes propriétés pharmacologiques ; ils sont en particulier des antagonistes de l'adostérone et augmentent la diurèse hydrosodée avec conservation du potassium organique.

Les produits de formule (I) offrent de plus l'avantage de ne présenter que très peu d'effets hormonaux secondaires.

Des tests effectués sur récepteur et in vivo ont, en particulier, montré que les produits de formule (I) sont moins anti-androgènes que des produits anti-aldostérone décrits précédemment.

Les produits de formule (I) peuvent donc être utilisés avantageusement pour lutter, notamment, contre l'hypertension artérielle et les insuffisances cardiaques.

L'invention a donc pour objet, à titre de médicaments, les produits de formule I telle que décrite ci-dessus.

L'invention a plus particulièrement pour objet, à titre de médicaments les produits de formule générale I dans laquelle R est choisi dans le groupe formé par un atome d'hydrogène, un radical alkyle ayant de 1 à 3 atomes de carbone, un radical hydroxyméthyle et un radical phényle, $R_1$ est choisi dans le groupe formé par un atome d'hydrogène, un radical alkyle ayant de 1 à 4 atomes de carbone et un radical acétylthio et X et Y sont tels que <u>soit</u> X représente un radical hydroxy et Y représente ou bien un radical $CH_2CH_2CO_2M'$ dans lequel M' représente un atome d'hydrogène ou un atome de métal alcalin ou bien Y représente un atome d'hydrogène <u>soit</u> X et Y représentent un radical :

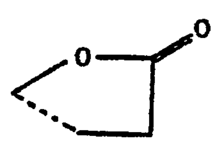

L'invention a plus spécialement pour objet, à titre de médicaments les produits de formule générale I dont les noms suivent :

- la gamma-lactone de l'acide 10 béta-éthynyl 17 béta-hydroxy 3-oxo 19-nor 17 alpha-pregn 4-èn 21-carboxylique,
- la 17 béta-hydroxy 10 béta-(1-propynyl) estr-4-èn 3-one,
- la gamma-lactone de l'acide 17 béta-hydroxy 3-oxo 10 béta-(1-propynyl) 19-nor 17 alpha-pregn 4-èn 21-carboxylique.

La posologie utile varie en fontion de l'affection à traiter et de la voie d'administration ; elle peut aller par exemple de 5 mg à 500 mg et de préférence de 10 à 200 mg par jour chez l'adulte par voie orale pour le produit de l'exemple 1.

Les composés de formule (I) sont utilisés par voie buccale, rectale, transcutanée, ou intraveineuse. Ils peuvent être prescrits sous forme de comprimés, de comprimés enrobés, de cachets, de capsules, de granulés, d'émulsions, de sirops, de suppositoires et de préparations injectables.

L'invention a donc également pour objet les compositions pharmaceutiques renfermant comme principe actif au moins un composé de formule (I). Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine aminale ou végétale, les dérivés paraffiniques, les glycols, les divers agents mouillants, dispersants ou émulsifiants, les conservateurs.

Les exemples suivants illustrent l'invention sans toutefois la limiter :

**Exemple 1** : gamma-lactone de l'acide 10 béta-éthynl 17 béta-hydroxy 3-oxo 19-nor 17 alpha-pregn 4-èn 21-carboxylique.

a/. Lactonisation

On introduit sous atmosphère inerte 20,8 cm3 de n-butyllithium dans l'hexane 1,65 N, à -50°C, dans 21 cm3 de tétrahydrofuranne, ajoute en 15 minutes à -50°C, 3,52 cm3 de N, N, N', N'-tétraméthylphosphoro diamidate d'allyle en solution dans 15 cm3 de tétrahydrofuranne, agite la solution orangée résultant pendant 1 heure à -30°C, ajoute en 10 minutes environ à -30°C 1,55 g de 3,3-(1,2-éthanediyl) acétal cyclique de 5 alpha-hydroxy 10 béta-éthynyl estran-3,17-dione en solution dans 30 cm3 de tétrahydrofuranne, laisse revenir à 20°C, agite pendant 1 heure et 30 minutes à 20°C, introduit goutte à goutte à +3°C 30cm3 de solution aqueuse 2N d'acide chlorhydrique, agite pendant 30 minutes à température ambiante, extrait à l'acétate d'éthyle, lave à l'eau salée, sèche, concentre à sec par distillation sous pression réduite et obtient 2,7 g de produit attendu

(A).

b/. Décétalisation et déshydratation

On dissout à 20°C sous atmosphère inerte les 2,7 g de

(A)

obtenus ci-dessus dans 27 cm3 d'éthanol, ajoute 5,4 cm3 de solution aqueuse 6 N d'acide chlorhydrique, agite à 50°C pendant 1 heure et 45 minutes. On concentre à sec souspression réduite, ajoute de l'eau salée, extrait au chlorure de méthylène, lave à l'eau, sèche, concentre à sec par distillation sous pression réduite, chromatographie le résidu (1,5 g) sur silice en éluant par le mélange éther de pétrole-acétate d'éthyle (6/4) et obtient 900 mg de gamma-lactone de l'acide 10 béta-éthynyl 17 béta-hydroxy 3-oxo 19-nor 17 alpha-prégn 4-ène 21-carboxylique

(3)

F = 136°C (peu net), ainsi que 260 mg de 10 béta-éthynyl estr 4-èn 3,17-dione F = 206°C

(1)

et 110 mg de gamma-lactone de l'acide 10 béta-éthynyl 17 alpha-hydroxy 3-oxo 19-nor prégn-4-èn 21-carboxylique F= 265°C

(2).

Contrôle du produit (1)

Spectre IR (chloroforme)

absence d'OH ; -C≡CH à $3305^{cm-1}$ (f) et 2100 cm-1 (f)
3-one: $1672^{cm-1}$ et $1645^{cm-1}$; 17-one $1735^{cm-1}$,
delta 4 $1623^{cm-1}$; c=c de f. $867^{cm-1}$.

Spectre de RMN (deutérochloroforme)

Pic à 0,96 ppm attribué aux hydrogènes du 18 Me;
Pic à 2,29 ppm attribué à l'hydrogène de C≡CH;
Pic à 5,83 ppm attribué à $H_4$.

Contrôle du produit (2)

Spectre IR (chloroforme)

C≡CH à $3305^{cm-1}$; gamma-lactone à $1763^{cm-1}$;
$delta_4$, 3-one $1671^{cm-1}$, 1624 et $866^{cm-1}$.

Spectre de RMN (deutérochloroforme)

Pic à 0,84 ppm attribué aux hydrogènes du 18 Me;
Pic à 2,27 ppm attribué à l'hydrogène de C≡CH;
Pic à 5,28 ppm attribué à $H_4$.
Les autres protons de 0,9 à 2,8 ppm.

Contrôles du produit attendu (3)

On purifie pour analyse le produit (3) par cristallisation dans un mélange de chlorure de méthylène et d'éther isopropylique. On obtient 500 mg de produit cristallisé F=136°C (peu net).

Analyse $C_{29}$ $H_{28}$ $O_3$ (352,45) Calculé : C% 78,37 H% 8,01
Trouvé C% 78,2 H% 8,1
(après séchage à 135°C)
/alpha/$^{20}$ : +155°,5 (C = 1% chloroforme)

Spectre $I_R^D$ (chloroforme)

≡CH à $3305^{cm-1}$; gamma-lactone à $1764^{cm1}$
delta 4 3-one à $1672^{cm-1}$, $1624^{cm-1}$, $867^{cm-1}$,

Spectre de UV (éthanol)

Max. 235 nm ε = 16700
Max. 314 nm ε = 1700

Spectre de RMN (deutérochloroforme)

Pic à 1,08 ppm attribué aux hydrogènes du 18 Me ;
Pic à 2,30 ppm attribué à l'hydrogène de C≡CH ;
Pic à ∿ 5,81 ppm attribué à l'hydrogène $H_4$.
Pics de 0,9 à 2,8 ppm, les autres protons.

Préparation : la 3,3-(1,2-éthanediyl) acétal cyclique de 10 béta- éthynyl 5 alpha-hydroxy estran 3,17-dione a été préparée comme suit :

On dissout à 20°C sous atmosphère inerte 1,9 g de 1,2-éthanediyl acétal cyclique de 10 béta-éthynyl 5 alpha 17 béta-dihydroxy estran 3-one décrite dans le brevet européen EP. 0.023.856 dans 60 cm3 d'acétone, ajoute goutte à goutte en 20 minutes environ, à +5cm3 de réactif de Bowers, agite 40 minutes à +5°C, ajoute 2 cm3 d'isopropanol, 10 cm3 d'acétate d'éthyle, lave au thiosulfate de sodium, à l'eau salée, sèche, concentre à sec par distillation sous pression réduite, cristallise les 1,92 g de produit brut ainsi obtenu dans un mélange de chlorure de méthylène et d'éther isopropylique et obtient 1,55 g de produit recherché F = 207°C

| Analyse $C_{22}H_{30}O_4$ (358,48) | | | | |
|---|---|---|---|---|
| Calculé | C% | 73,71 | H% | 8,43 |
| Trouvé | | 74,0 | | 8,6 |

Spectre IR (chloroforme)

OH en 5 à 3480 $cm^{-1}$; C≡CH à 3305 $cm^{-1}$; C=0 à 1731 $cm^{-1}$

Spectre de RMN (deutérochloroforme)

Pic à 0,92 ppm attribué aux hydrogènes du méthyle 18;
Pic à 8,25 ppm attribué à l'hydrogène de l'éthynle;

Pics à 3,99 ppm attribuées aux hydrogènes de méthylènes de 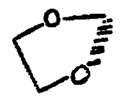

Pics à 4,27 ppm attribué à l'hydrogène de l'hydroxyle en position 5.

**Exemple 2** : 17 béta-hydroxy 10 béta-(1-propynyl) estr-4-èn 3-one

On met en suspension, sous atomosphère inerte, 1 g de 1,2-éthane diyl acétal cyclique de 5 alpha-hydroxy 10 béta-(1-propynyl) 17 béta-(2H-tétrahydropyrannyl-2-oxy) estran 3-one dans 10 cm3 d'éthanol et 2 cm3 de solution aqueuse d'acide chlorhydrique 6N, chauffe à 50°C, obtient une solution au bout de 15 minutes environ, agite pendant 5 heures et 15 minutes à 50°C, élimine l'éthanol par distillation sous pression réduite, reprend le résidu à l'eau, extrait au chlorure de méthylène, lave à l'eau, sèche, concentre à sec par distillation sous pression réduite, chromatographie le résidu (840 mg) sur silice en éluant par un mélange cyclohexane-acétate d'éthyle (1/1) et obtient 570 mg de produit recherché F = 172°C.

Ce produit est cristallisé pour microanalyse dans un mélange de chlorure de méthylène et d'éther isopropylique. On obtient à partir des 570 mg, 520 mg de produit recherché F = 172°C.

| Analyse $C_{21}H_{28}O_2$ (312,45) | | | | |
|---|---|---|---|---|
| Calculé | C% | 78,1 | H% | 8,8 |
| Trouvé | | 78,3 | | 9,1 |

Spectre IR (chloroforme)

OH à 3612 $cm^{-1}$; cétone conjugée à 1667 $cm^{-1}$;
-C=C- à 1622 $cm^{-1}$;

Spectre UV (éthanol)

Max. 235 nm ε = 15700

Spectre de RMN (deutérochloroforme)

Pic à 0,84 ppm attribué aux hydrogènes du méthyle 18 ;
Pic à 1,88 ppm attribué aux hydrogènes du méthyle du propylene;
Pic à 3,66 ppm attribué à l'hydrogène en 17;
Pic à 5,78 ppm attribué à hydrogène en position 4.

<u>Préparation: Le 1,2-éthanediyl acétal cylique de 5 alpha-hydroxy 10 béta-(1-propynyl) 17 béta-(2H-tétrahydropyrannyl 2-oxy) estran 3-one a été préparé comme suit:</u>

<u>Stade A</u> : 1,2-éthanediyl acétal cyclique de 10 béta-éthynyl 5 alpha-hydroxy 17 béta-(2H-tétrahydropyrannyl 2-oxy) estran 3-one.

On mélange à 20°C 3,75 g de 1,2-éthanediyl acétal cyclique de 10 béta-éthynl 5 alpha,17 béta-dihydroxy estran-3-one, 37 $cm^3$ de tétrahydrofuranne, 10 $cm^3$ de dihydropyranne, 0,2 $cm^3$ de $POCl_3$, agite la solution jaune résultante pendant 1 heure et 30 minutes à 20°C, verse le mélange réactionnel sur 50 $cm^3$ d'eau, extrait au chlorure de méthylène, lave à l'eau, sèche, concentre à sec par distillation sur pression réduite, chromatographie le résidu (6,15 g) sur silice en éluant par un mélange d'éther de pétrole et d'acétate d'éthyle (8/2) + 1‰ de triéthylamine et obtient 4,45 g de produit recherché F $\sim$ 100°C.

<u>Spectre IR</u> (chloroforme)

absence d'OH en 17 ; OH en 5 à 3485 $^{cm-1}$;
-C=CH à 3305 $^{cm-1}$ (f) et 2100 cm-1 (f)

<u>Spectre de RMN</u> (deutérochloroforme + 1 goutte de $C_5D_5M$)

Pic à 0,82 et 0,83 ppm attribué aux hydrogènes du méthyle 18;
Pic à 2,24 ppm attribué à l'hydrogène de léthynle;
Pic à 3,33 ppm attribué à 4,0 ppm aux hydrogènes

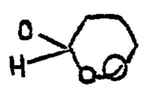

Pic à 3,94 ppm attribué aux hydrogènes des méthylènes de

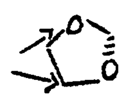

Pic à 4,23 ppm attribué à hydrogène de l'hydroxyle en 5;
Pic à 4,64 ppm attribué à hydrogène

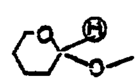

<u>Stade B</u> : 1,2-éthanediyl acétalcyclique de 5 alpha-hydroxy 10 béta-(1-propynyl) 17 béta-(2H-tétrahydropyrannyl-2-oxy) estran 3-one.

On dissout à 20°C, sous atmosphère inerte 4,42 g de 1,2-éthanediyl acétal cyclique de 5 alpha-hydroxy 10 béta-éthynyl 17 béta- 2H-tétrahydropyrannyl 2-oxy estran-3-one dans 40 $cm^3$ de tétrahydrofuranne, ajoute 13,7 $cm^3$ de n-butyllithium 1,6 M dans le tétrahydrofuranne, ramène à 20°C, ajoute 3,1 $cm^3$ d'iodure de méthyle, le mélange réactionnel passe de 20 à 33°C, on obtient une solution incolore, agite pendant 45 minutes, puis introduit à +3°C 20 $cm^3$ de solution aqueuse de chlorure d'ammonium, décante, extrait à l'acétate d'ethyle, lave à l'eau salée, sèche, concentre à sec en distillant sous pression réduite, chromatographie le résidu (4,47 g) sur silice en éluant par le mélange éther de pétrole-acétate d'éthyle (8-2) + 1‰ de triétylamine et obtient 3,9 g de produit recherché F = 113°C

<u>Spectre IR</u> (chloroforme)

OH en 5 à 3485 $^{cm-1}$

<u>Spectre de RMN</u> (deutérochloroforme)

Pic à 0,8 et 0,82 ppm attribué aux hydrogènes du méthyle 18;
Pic à 1,85 ppm attribué au méthyle du propényle
Pic à 3,33 ppm et 4 ppm attribuées aux hydrogènes

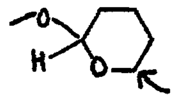

Pic à 4,64 ppm attribué à l'hydrogène

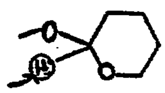

**Exemple 3** : gamma-lactone de l'acide 17 béta-hydroxy 3-oxo 10 béta-(1-propynyl) 19-nor 17 alpha-pregn 4-ène 21-carboxylique.

a/. Lactonisation

Dans 12 $cm^3$ de n-butyllithium 1,65 N en solution dans l'hexane, on introduit à -50°C 12 $cm^3$ de tétrahydrofuranne, puis en 15 minutes environ à -50°C 2,06 $cm^3$ de N,N,N',N' tétraméthylphosphoramidate d'allyle en solution dans 8 $cm^3$ de tétrahydrofuranne, agite à -30°C pendant 1 heure, ajoute en 10 minutes environ à -30°C 880 mg de 3,3-(1,2-éthanediyl) acétalcyclique de 5 alpha-hydroxy 10 béta-(1-propynyl) estran 3,17-dione, laisse revenir à 20°C, agite pendant 1heure à 20°C, introduit à +3°C 20 $cm^3$ de solution aqueuse 2N d'acide chlorhydrique, agite à 20°C pendant environ 30 minutes, décante, lave à l'eau salée, extrait à l'acétate d'éthyle, lave à l'eau, sèche, concentre à sec par distillation sous pression réduite et obtient 2 g de résidu sec.

b/. Décétalisation et déshydratation

On dissout à 20°C, sous atmosphère inerte, les 2 g isolés ci-dessus dans 40 $cm^3$ d'éthanol, ajoute 4 $cm^3$ de solution aqueuse 6 N d'acide chlorhydrique, chauffe à 60°C pendant 3 heures et 30 minutes, distille l'ethanol sous pression réduite, reprend le résidu au chlorure de méthylène, lave à l'eau, sèche et concentre à sec par distillation sous pression réduite, purifie le résidu (1,2 g) par chromatographie sur silice en éluant par un mélange d'éther de pétrole et d'acétate d'éthyle 6/4, puis par un mélange d'éther de pétrole et d'acétate d'éthyle 1/1 et obtient 415 mg de gamma-lactone de l'acide 17 béta-hydroxy 3-oxo 10 béta-(1-propynyl) 19-nor 17 alpha-pregn 4-ène 21-carboxylique brut attendu, F=234°C.

Ce produit est cristallisé dans un mélange de chlorure de méthylène et d'éther isopropylique pour obtenir 361 mg de produit pur F=235°C.

| Analyse $C_{24}H_{30}O_3$ (366,51) | | | | |
|---|---|---|---|---|
| Calculé | C% | 78,65 | H% | 8,25 |
| Trouvé | | 78,5 | | 8,4 |

Spectre IR (chloroforme)

gamma-lactone C=O à 1764$^{cm-1}$, 3 céto delta 4 à 1669, CN 1623$^{cm-1}$

Spectre de RMN (deutérochloroforme)

Pic à 1,03 ppm attribué à 18 Me;
Pic à 1,88 ppm attribué à $CH_3$-C=C;
Pic à 5,79 ppm attribué à $H_4$.
On abtient en outre
– 290 mg de 10 béta-(1-propynyl) estr 4-èn 3,17-dione, F=212°C

Spectre IR (chloroforme)

17 céto à 1733$^{cm-1}$; 3 céto delta 4 1660–1622$^{cm-1}$

Spectre de RMN (deutérochloroforme)

Pic à 0,96 ppm attribué à 18 Me;
Pic à 1,87 ppm attribué à =C-Me;
Pic à 5,69 ppm attribué à $H_4$
ainsi que 30 mg de gamma-lactone de l'acide 17 alpha-hydroxy 3-oxo 10 béta-(1-propynyl) 19-nor-pregn 4-èn 21-carboxylique, F = 188°C

Spectre IR (chloroforme)

C = O de gamma-lactone 1762$^{cm-1}$, 3 céto delta 4 : 1669 et 1622$^{cm-1}$.

Spectre de RMN (deutérochloroforme)

Pic à 0,83 ppm attribué à 18 Me ;
Pic à 1,88 ppm attribué à $CH_3$-C=C ;
Pic à 5,79 ppm attribué à $H_4$.

Préparation : le 3,3-(1,2-éthanediyl) acétalcyclique de 5 alpha-hydroxy 10 béta-(1 propynyl)-estran 3,17-dione a été préparé comme suit :

Stade A: 3,3-(1,2 éthane diy)l acétal cyclique de 5 alpha,17 béta-dihydroxy 10 béta-(1-propynyl) estran 3-one

On dissout à 20°C, sous atmosphère inerte 2,2 g de 1,2-éthanediyl acétal cyclique de 5 alpha-hydroxy 10 béta-(1-propynyl) 17 béta-(2H-tétrahydropyrannyl 2-oxy) estran-3-one, dans 44 cm³ d'acide acétique, ajoute 11 cm³ d'eau, agite pendant 35 minutes à 20°C, élimine l'acide acétique par distillation sous pression réduite, ajoute du bicarbonate de sodium en solution aqueuse pour neutraliser, extrait à l'acétate d'éthyle, lave à l'eau salée, sèche, concentre à sec par distillation sous pression réduite. Le résidu (2,3 g) est chromatographié sur silice en éluant par des mélanges de cyclohexane-d'acétate d'éthyle (7/3) puis (1/1) et recueille 700 mg de produit recherché (II), ainsi que :
– 715 mg de produit de départ (I) ;
– 225 mg de 5 alpha-hydroxy 10 béta-(1-propynyl) 17 béta-tétrahydropyrannyloxy estran 3-one (IV) et :
– 200 mg de 5 alpha, 17 béta-dihydroxy 10 béta-(1-propynyl) estran 3-one (III).

Contrôle du produit II (produit recherché)

Spectre IR (chloroforme)

OH en 17 à 3609$^{cm-1}$; OH en 5 à 3480$^{cm-1}$.

Spectre de RMN (deutérochloroforme + 1 goutte de $C_5O_5N$)

Pic à 0,8 ppm attribué aux hydrogènes du méthyle 18;
Pic à 1,86 ppm attribué aux hydrogènes du méthyle du propynyle;
Pic à 2,97 ppm et 4,2 ppm attribuées aux hydrogènes des hydroxyles en 5 et en 17;
Pics à 3,58-3,66-3,75 ppm attribués à l'hydrogène en 17.

Contrôle du produit (IV) :

Spectre IR (chloroforme)

OH : 3305$^{cm-1}$ (libre) + un peu d'associé;
C=O : 1709$^{cm-1}$;

Spectre de RMN (deutérochloroforme)

Pic à 0,83–0,85 ppm attribués aux hydrogènes du méthyle 18;
Pic à 1,91 ppm attribué aux hydrogènes du méthyle du propynyle;
Pic de 3,33 à 4,11 ppm attribués aux hydrogènes

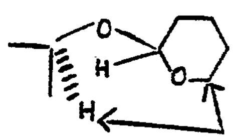

Pic de 4,64 ppm attribué à hydrogène

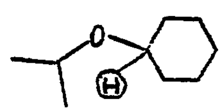

Contrôle du produit (III)

Spectre IR (chloroforme)

OH libre $3609^{cm-1}$ + associé;
C = O $1709^{cm-1}$.

Spectre de RMN (deutérochloroforme)

Pic à 0,83 ppm attribué aux hydrogènes du méthyle 18;
Pic à 1,93 ppm attribué aux hydrogènes du méthyle du propynyle;
Pic à 3,59-3,67-3,74 ppm attribués à l'hydrogène en 17.

Stade B : 3,3-(1,2-éthanediyl) acétal cyclique de 5 alpha-hydroxy 10-béta-(1-propynyl) estran 3,17-dione.

On dissout à 20°C, sous atmosphère inerte 990 mg de 1,2-éthanediyl acétal cyclique de 5 alpha, 17 béta-dihydroxy 10 béta-(1-propynyl) estran 3-one dans 30 $cm^3$ d'acétone, ajoute goutte à goutte, à +3°C, 0,92 $cm^3$ de réactif de Bowers, agite pendant 1 heure 30 à + 3°C, verse le mélange réactionnel sur du bicarbonate de sodium, extrait à l'acétate d'éthyle, lave à l'eau salée, sèche, concentre à sec par distillation sous pression réduite, obtient 1 g de résidu sec, que l'on chromatographie sur silice en éluant par un mélange de cyclohexane et d'acétate d'éthyle (7/3) à 1% de triéthylamine et obtient 860 mg de produit recherché F=172°C.
Un échantillon est cristallisé pour analyse dans un mélange de chlorure de méthylène et d'éther isopropylique. A partir de 125 mg, on obtient 85 mg, F = 172°C.

| Analyse | | | | |
|---|---|---|---|---|
| Calculé | C% | 74,16 | H% | 8,66 |
| Trouvé | | 74,1 | | 8,7 |

Spectre IR (chloroforme)

OH en 5 $3488^{cm-1}$; 1-céto $1731^{cm-1}$;

Spectre de RMN (deutérochloroforme)

Pic à 0,91 ppm attribué aux hydrogènes du méthyle 18;
Pic à 1,85 ppm attribué aux hydrogènes du méthyle du propynyle;
Pic à 3,98 ppm attribué aux hydrogènes des méthylènes de

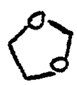

Pic à 4,22 ppm attribué à l'hydrogène de l'hydroxyle en position 5.

**Exemple 4** : Exemple de composition pharmaceutique:

On a préparé des comprimés à 50 mg de produit de l'exemple 1 comme principe actif.

| | |
|---|---|
| Produit de l'exemple 1 | 50 mg |
| Excipient (talc, amidon, stéarate de magnésium). | |

**ETUDE PHARMACOLOGIQUE DES PRODUITS DE L'INVENTION.**

Etude de l'activité antialdostérone sur le récepteur minéralocorticoïde du rein de rat :

Des rats mâles Sprague-Dawley EOPS, pesant 140 à 160 g, surrénalectomisés depuis 4 à 6 jours sont sacrifiés et leurs reins sont perfusés in situ avec 50 ml d'un tampon Tris 10mM-Saccharose 0,25 M, HCl pH 7,4. Les reins sont ensuite prélevés, décapsulés et homogénéisés à 0°C à l'aide d'un Potter polytétrafluoroéthylène-verre (1 g de tissu pour 3 ml de tampon). L'homogénat est centrifugé pendant 10 minutes à 800 g, à 0°C.

Afin d'éliminer la fixation de l'aldostérone tritiée sur le récepteur glucocorticoïde, le 11 béta, 17 béta-dihydroxy 17 alpha-(1-propynyl) androsta 1,4,6-trien 3-one stéroide se fixant uniquement sur le récepteur glucocorticoïde est additionné au surnageant à la concentration finale de $10^{-6}$M. Ce surnageant est ultracentrifugé à 105 000 g pendant 60 minutes à 0°C. Des aliquotes du surnageant ainsi obtenues sont incubées à 0°C avec une concentration constante (T) d'aldostérone tritiée en présence de concentrations croissantes (0-2500. $10^{-9}$M) d'aldostérone froide ou du produit froid à étudier. Après un temps (t) d'incubation, la concentration d'aldostérone tritiée liée (B) est mesurée par la technique d'adsorption au charbon-dextran.

<u>Calcul de l'affinité relative de liaison</u> :

Le calcul de l'affinité relative de liaison (ARL) est effectué comme suit :
On trace les 2 courbes suivantes : le pourcentage de l'hormone tritiée liée

$$\frac{B}{T}$$

en fonction du logarithme de la concentration de l'hormone de référence froide et

$$\frac{B}{T}$$

en fonction du logarithme de la concentration du produit froid testé.
On détermine la droite d'équation

$$I_{50} = \left(\frac{B}{T}\max + \frac{B}{T}\min\right)/2$$

$$\frac{B}{T}\max =$$

Pourcentage de l'hormone tritiée liée pour une incubation de cette hormone tritiée à la concentration (T).

$$\frac{B}{T}\min =$$

Pourcentage de l'hormone tritiée liée pour une incubation de cette hormone tritiée à la concentration (T) en présence d'un grand excès d'hormone froide (2500.$10^{-9}$M).
Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.
L'affinité relative de liaison (ARL) du produit testé est déterminée par l'équation :

$$ARL = 100 \frac{(CH)}{(CX)}$$

Les résultats obtenus sont les suivants :

| Temps d'incubation à O ¤ C<br>Produit l'exemple | 1H | 24 H |
|---|---|---|
| 1 | 125 | 65 |
| 3 | 50 | 43 |

**Revendications**

1. Les produits de formule générale I:

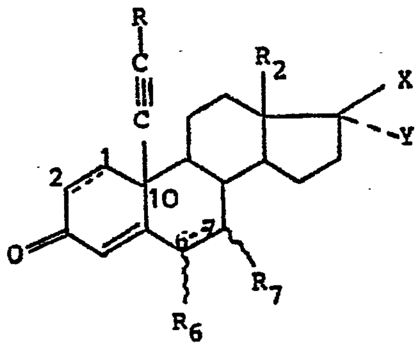

(I)

dans laquelle R représente:
- soit un atome d'hydrogène,
- soit un radical alkyle, alkényle ou alkynyle ayant au plus 8 atomes de carbone, éventuellement substitué par un radical choisi parmi les radicaux hydroxyle, carboxy, méthoxycarbonyle, éthoxycarbonyle, amino, tritylamino, chloroacétylamino, trifluoroacétylamino, trichloroéthoxycarbonylamino, méthylamino, diméthylamino ou halogène,
- soit un radical aryle ou aralkyle éventuellement substitué par un radical choisi parmi les radicaux hydroxyle, carboxy, méthoxycarbonyle, éthoxycarbonyle, amino, méthylamino, diméthylamino, méthyle méthoxy ou méthylthio,
- soit un radical alkoxy, carboxy méthoxycarbonyle, éthoxycarbonyle ou dialkylamino,
- soit un atome d'halogène,
- soit un radical trialkylsilyle,

$R_6$ et $R_7$ sont tels que, soit $R_6$ et $R_7$ forment avec les carbones qui les portent un radical cyclopropyle, soit $R_6$ représente un atome d'hydrogène et $R_7$ représente le radical $R_1$, $R_1$ représentant:
- soit un atome d'hydrogène,
- soit un radical alkyle, alkényle ou alkynyle ayant au plus 6 atomes de carbone, éventuellement substitué par un radical choisi parmi les radicaux hydroxyle, carboxy, méthoxycarbonyle, éthoxycarbonyle, amino, tritylamino, chloroacétylamino, trifluoroacétylamino, trichloroéthoxycarbonylamino, méthylamino, diméthylamino ou halogène,
- soit un radical acétylthio,

$R_2$ représente un radical méthyle ou éthyle,

X et Y sont tels que:
- soit X représente un radical hydroxyle, acétyloxy, propionyloxy, méthoxy ou éthoxy et Y représente ou bien un radical $-CH_2CH_2CO_2M$ dans lequel M représente un atome d'hydrogène, un atome de métal alcalin ou un radical ammonium,
ou bien un radical $-CH_2CH_2-CH_2OH$
- soit X et Y représentent ensemble un radical:

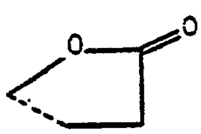

ou

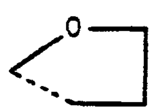

- soit X et Y représentent ensemble un radical

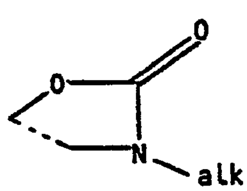

dans lequel alk représente un radical alkyle renfermant au plus 8 atomes de carbone,
- soit X et Y représentent ensemble un radical

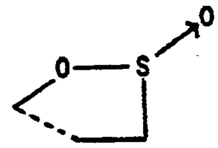

ou X représente un radical hydroxyle et Y un radical

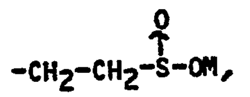

M étant défini comme ci-dessus,

– soit X représente un radical hydroxyle, acétyloxy, propionyloxy, méthoxy ou éthoxy et Y représente, ou bien un atome d'hydrogène ou bien Y représente $R_4$, $R_4$ représentant un radical alkyle, alkényle ou alkynyle ayant au plus 6 atomes de carbone,

les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle,

les traits ondulés en position 6 et 7 indiquent que les substituants $R_6$ et $R_7$ peuvent se trouver dans l'une des positions possibles alpha ou béta, étant entendu que R ne peut pas représenter un atome d'hydrogène lorsque $R_6$ et $R_7$ représentent chacun un atome d'hydrogène, $R_2$ représente un radical méthyle, X représente un radical hydroxy, acétyloxy, propionyloxy, méthoxy ou éthoxy et Y représente un atome d'hydrogène, ou $R_4$ et les traits pointillés en position 1 (2) et 6 (7) ne représentent pas ensemble une seconde liaison entre les carbones qui les portent.

2. Les produits de formule générale (I), telle que définie à la revendication 1, répondant à la formule $I_1$:

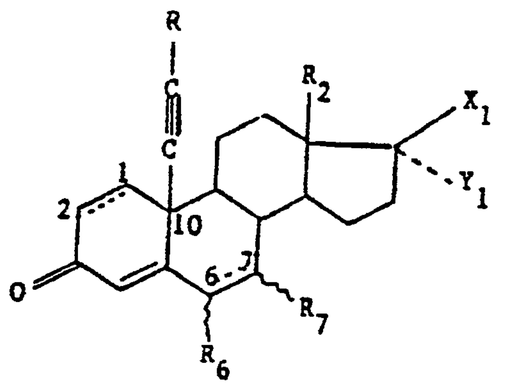 ($I_1$)

dans laquelle R, $R_6$, $R_7$, $R_2$, les traits pointillés en 1 (2) et 6 (7) et les traits ondulés en 6 et 7 ont les significations indiquées à la revendication 1,

$X_1$ et $Y_1$ sont tels que:

– soit $X_1$ représente un radical hydroxyle, acétyloxy, propionyloxy, méthoxy ou éthoxy, et $Y_1$ représente ou bien un radical $-CH_2CH_2CO_2M$ dans lequel M représente un atome d'hydrogène, un atome de métal alcalin ou un radical ammonium, ou bien un radical $-CH_2CH_2-CH_2OH$,

– soit $X_1$ et $Y_1$ représentent ensemble un radical:

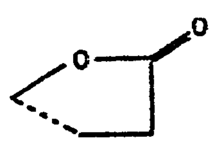 ou 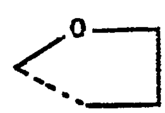

– soit $X_1$ représente un radical hydroxyle, acétyloxy, propionyloxy, méthoxy ou éthoxy, et $Y_1$ représente, ou bien un atome d'hydrogène ou bien $Y_1$ représente $R_4$, $R_4$ représentant un radical alkyle, alkényle ou alkynyle ayant au plus 6 atomes de carbone, étant entendu que R ne peut pas représenter un atome d'hydrogène lorsque $R_6$ et $R_7$ représentent chacun un atome d'hydrogène, $R_2$ représente un radical méthyle, $X_1$ représente un radical hydroxy, acétyloxy, propionyloxy, méthoxy ou éthoxy, $Y_1$ représente un atome d'hydrogène ou $R_4$ et les traits pointillés en position 1 (2) et 6 (7) ne représentent pas une seconde liaison entre les carbones qui les portent.

3. Les produits de formule générale (I), telle que définie à la revendication 1, répondant à la formule:

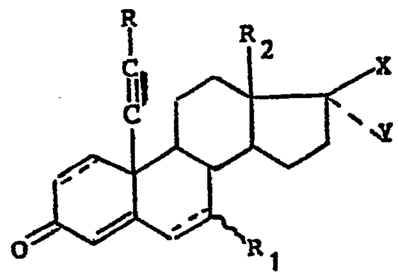

dans laquelle R, $R_1$, $R_2$, X, Y, les traits pointillés et les traits ondulés ont la signification indiquée à la revendication 1.

4. Les produits de formule I telle que définie à l'une quelconque des revendications 1 à 3, dans laquelle R est choisi dans le groupe formé par l'atome d'hydrogène, les radicaux alkyles ayant de 1 à 3 atomes de carbone, le radical hydroxyméthyle et le radical phényle.

5. Les produits de formule I telle que définie à l'une quelconque des revendications 1 à 4, dans laquelle le substituant $R_1$ est choisi dans le groupe formé par l'atome d'hydrogène, les radicaux alkyles ayant de 1 à 4 atomes de carbone et le radical acétylthio et X et Y sont tels que soit X représente un radical hydroxy et Y représente ou bien un radical $CH_2CH_2CO_2M'$ dans lequel M' représente un atome d'hydrogène ou atome de métal alcalin, ou bien Y représente un atome d'hydrogène,
– soit X et Y représentent un radical:

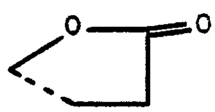

6. Les produits de formule générale (I), dont les noms suivent:
– la gamma-lactone de l'acide 10 béta-éthynyl 17 béta-hydroxy 3-oxo 19-nor 17 alpha-pregn 4-èn 21-carboxylique,
– la 17 béta-hydroxy 10 béta-(1-propynyl) estr-4-èn 3-one,
– la gamma-lactone de l'acide 17 béta-hydroxy 3-oxo 10 béta-(1-propynyl) 19-nor 17 alpha-pregn 4-èn 21-carboxylique.

7. Procédé de préparation des produits de formule I, telle que définie à la revendication 1, étant entendu que R ne peut pas représenter un atome d'hydrogène lorsque $R_6$ et $R_7$ représentent chacun un atome d'hydrogène, $R_2$ représente un radical méthyle, X représente un radical hydroxy, acétyloxy, propionyloxy, méthoxy ou éthoxy et Y représente un atome d'hydrogène ou $R_4$ et les traits pointillés en position 1 (2) et 6 (7) ne représentent pas ensemble une seconde liaison entre les carbones qui les portent, caractérisé en ce que l'on traite un produit de formule II:

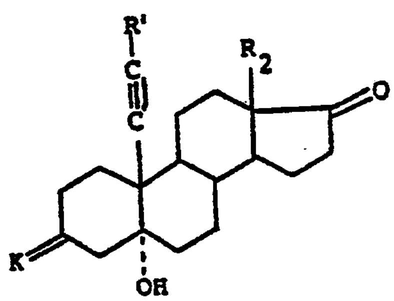

(II)

dans laquelle R' représente ou bien R, R ayant la signification indiquée à la revendication 1, ou bien R dans lequel les fonctions réactives sont protégées, $R_2$ a la signification indiquée à la revendication 1 et K représente un groupement protecteur de la fonction cétone,
I. soit d'abord par un halogénure de triméthylsulfonium en présence d'une base forte, puis par un dérivé métallique de l'acétonitrile et enfin par une base, puis par un acide,
soit d'abord par un réactif de formule:

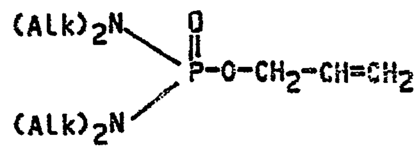

dans lequel Alk représente un radical alkyle ayant de 1 à 4 atomes de carbone en présence d'une base forte puis par un acide, pour obtenir un produit de formule $I_A$:

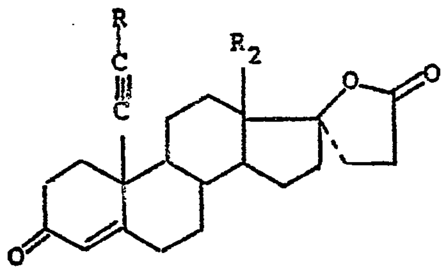

$(I_A)$

II. <u>soit</u> par un réactif de formule:

$$XMg-CH_2-CH_2-CH_2-OB$$

dans lequel X représente un atome d'halogène et B représente un groupement protecteur du radical hydroxyle ou un alcoolate de magnésium pour obtenir, après traitement par un acide, un produit de formule $I'_A$:

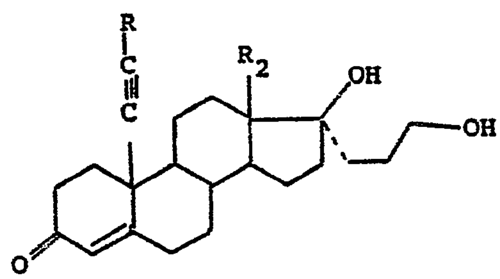

$(I'_A)$

produit de formule $I'_A$ que l'on traite éventuellement par un halogénure d'acide sulfonique en présence d'une base pour obtenir un produit de formule $I''_A$:

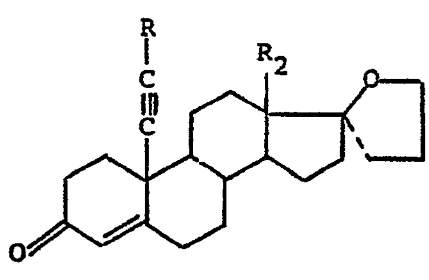

$(I''_A)$

III. <u>soit</u> par un réactif de réduction, puis par un acide, pour obtenir un produit de formule $I_{3A}$:

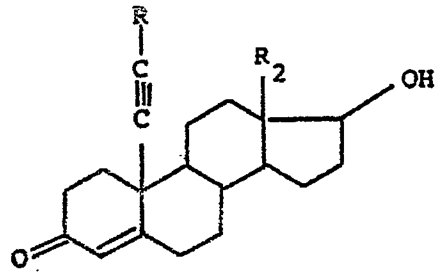

(I3A)

IV. soit par un organométallique $R_4MgX$, X représentant un atome d'halogène, puis par un acide, pour obtenir un produit de formule $I_{4A}$:

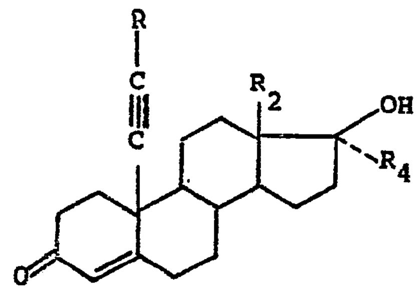

(I4A)

V. soit d'abord par un halogénure de triméthylsulfonium en présence d'une base forte, puis par une amine primaire de formule $H_2N$-alk, alk étant défini comme précédemment, en présence d'un acide puis par un chloroformiate d'alkyle, par une base forte et enfin par un acide, pour obtenir un produit de formule $I_{5A}$:

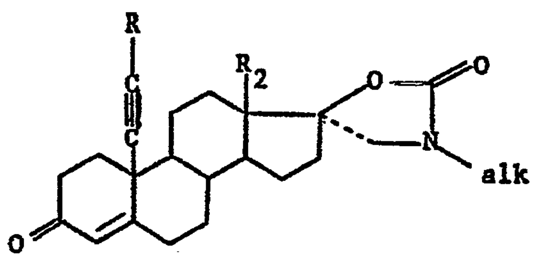

(I5A)

VI. soit d'abord par un halogénure de triméthylsulfonium en présence d'une base forte, puis par le méthyl terbutyl sulfoxyde en présence de n-butyl-lithium pour obtenir un produit de formule:

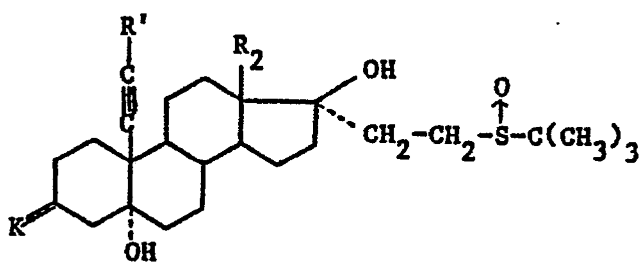

sous forme d'un mélange de deux diastéréoisomères au niveau de l'atome de soufre, sépare si désiré les deux diastéréoisomères, puis soumet soit leur mélange, soit chacun d'eux séparément, à l'action d'un acide, pour obtenir un produit de formule:

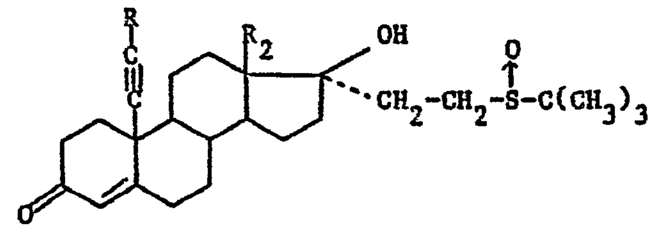

sous forme d'un mélange de deux diastéréoisomères ou d'un diastéréoisomère, sépare le cas échéant, les diastéréoisomères, puis soumet soit leur mélange, soit chacun d'eux séparément, à l'action du N-chloro ou du N-bromo succinimide, pour obtenir un produit de formule $I_{6A}$:

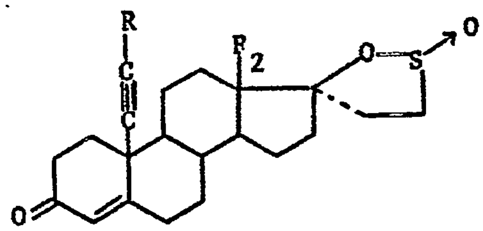

$I_{6A}$

et que, si désiré, l'on traite les produits de formules $I_A$, $I'_A$, $I''_A$, $I_{3A}$, $I_{4A}$, $I_{5A}$ et $I_{6A}$ par un orthoformiate d'alkyle en présence d'un acide, puis par un agent de déhydrogénation, pour obtenir les produits de formules $I_B$, $I'_B$, $I''_B$, $I_{3B}$, $I_{4B}$, $I_{5B}$ et $I_{6B}$ suivantes.

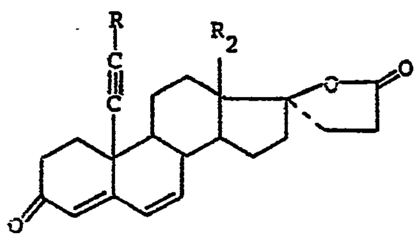

$I_B$

;

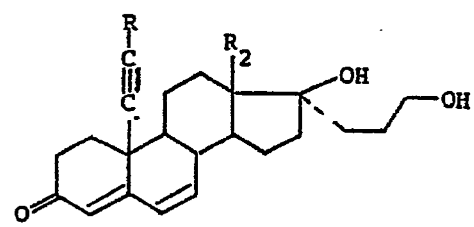

$I'_B$

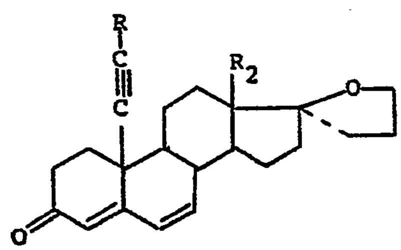

$I''_B$

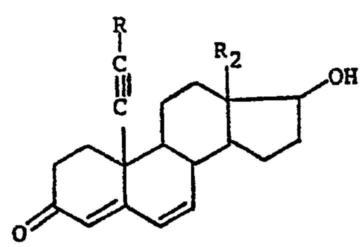

$I_{3B}$

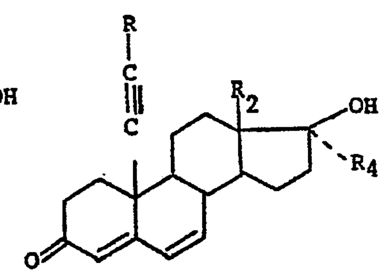

$I_{4B}$

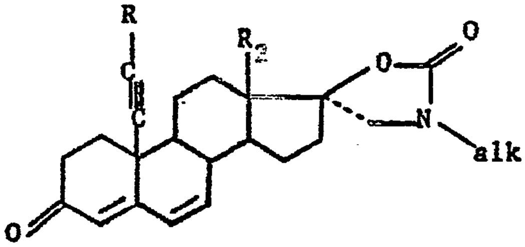

$I_{5B}$

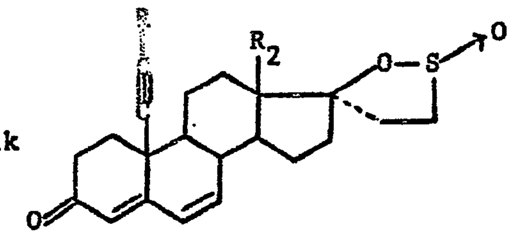

$I_{6B}$

produits de formules $I_B$, $I'_B$, $I''_B$, $I_{3B}$, $I_{4B}$, $I_{5B}$ et $I_{6B}$ que l'on traite si désiré:
– <u>ou bien</u> par un organo magnésien de formule $R_1MgX'$ éventuellement en présence d'un sel de cuivre, formule dans laquelle $R_1$ à la signification indiquée ci-dessus et X' représente un atome d'halogène,
– <u>ou bien</u> par un organo métallique de formule $(R_1)_2$ CuLi puis par un acide,
pour obtenir les produits de formules $I_C$, $I'_C$, $I''_C$, $I_{3C}$, $I_{4C}$, $I_{5C}$ et $I_{6C}$ suivantes:

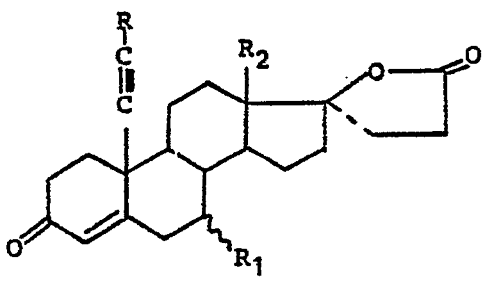

$I_C$

;

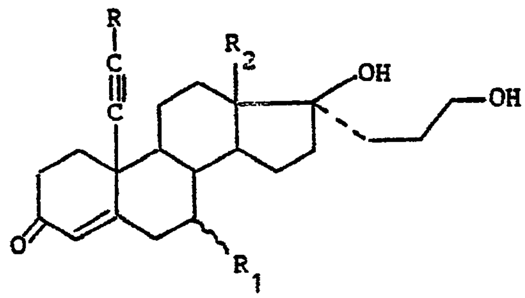

$I'_C$

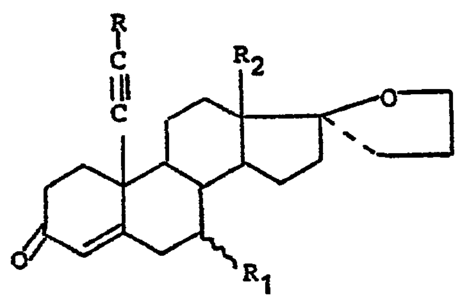

$I''_C$

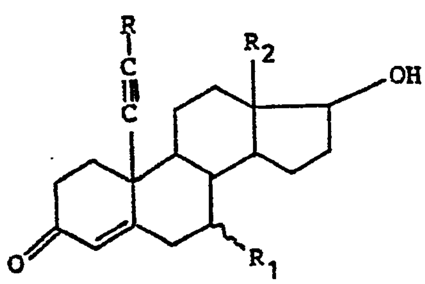

$I_{3C}$

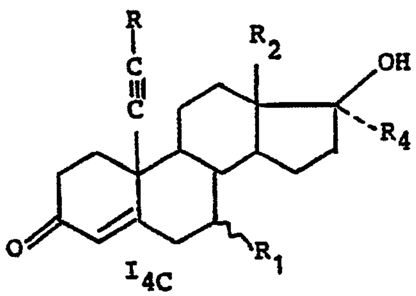

$I_{4C}$

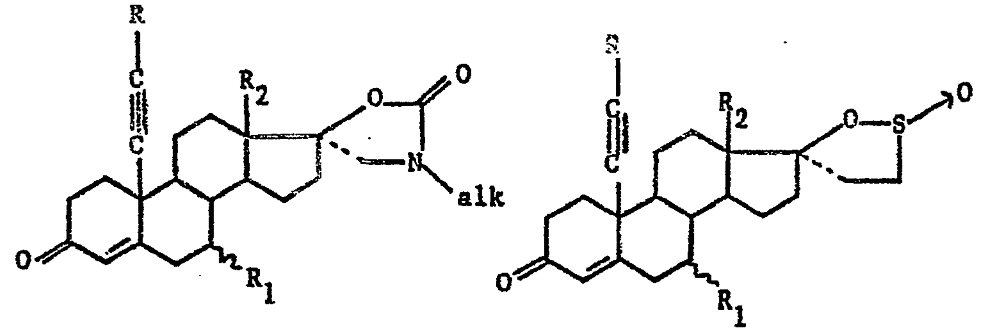

$I_{5C}$ $I_{6C}$

sous forme d'un mélange 7 alpha et 7 béta, mélange que, si désiré, l'on sépare et que, si désiré, l'on soumet les produits 7 béta à un réactif de déshydrogénation pour obtenir les produits delta 6 (7) correspondants:

– <u>ou bien</u> l'on soumet les produits de formules $I_B$, $I'_B$, $I''_B$, $I_{3B}$, $I_{4B}$, $I_{5B}$ et $I_{6B}$ à l'action d'un réactif choisi dans le groupe constitué par l'iodure de triméthylsulfonium et l'iodure de triméthylsulfoxonium en présence d'une base forte pour obtenir les produits de formules $I_D$, $I'_D$, $I''_D$, $I_{3D}$, $I_{4D}$, $I_{5D}$ et $I_{6D}$ suivantes:

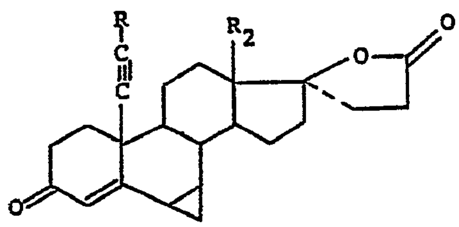

$I_D$

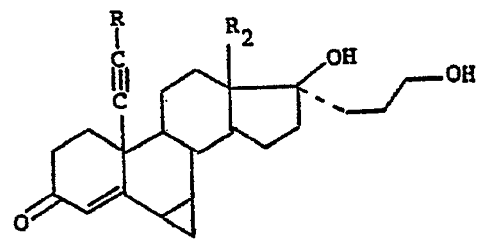

$I'_D$

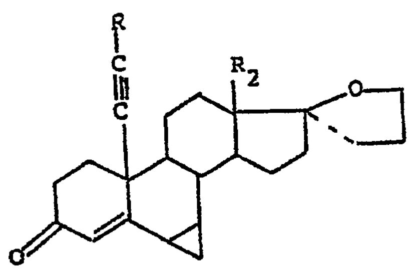

$I''_D$

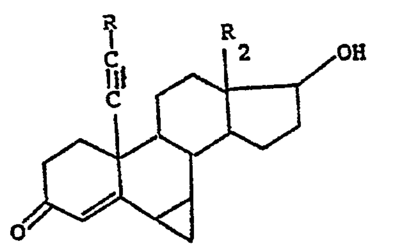

$I_{3D}$

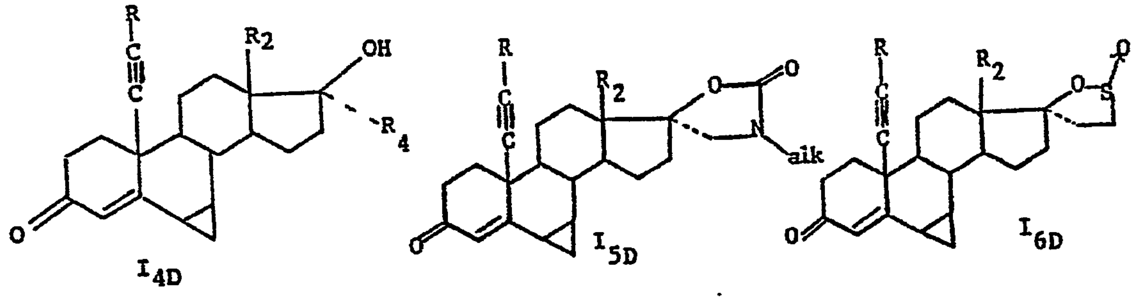

$I_{4D}$ $I_{5D}$ $I_{6D}$

sous forme d'un mélange 6 alpha, 7 alpha et 6 béta, 7 béta et sépare, si désiré, les isomères ainsi obtenus et que si désiré l'on traite les produits $I_A$, $I'_A$, $I''_A$, $I_{2A}$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$,

$I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$, par un réactif de déshydrogénation ou par un microorganisme susceptible de déshydrogéner la molécule en position 1 (2) pour obtenir les produits correspondants comportant une insaturation en position 1 (2) et que, si désiré, l'on traite les produits de formules $I_A$, $I_B$, $I_C$ et $I_D$ et les produits correspondants comportant une insaturation en position 1 (2) à l'aide d'un hydroxyde alcalin ou de l'ammoniaque pour obtenir les produits correspondants dans lesquels X représente un radical hydroxyle et Y représente un radical $-CH_2CH_2CO_2M'$ dans lequel M' représente un atome de métal alcalin ou un radical ammonium et, si désiré, soumet les produits ainsi obtenus à l'action d'un agent acide pour obtenir les produits dans lesquels X représente un radical hydroxyle et Y représente un radical $-CH_2CH_2CO_2H$, et que si désiré l'on traite les produits dans lesquels le substituant en position 10 comporte un substituant R du type hydroxyalkyle, par un tétra-bromure ou tétrachlorure de carbone, en présence de triphénylphosphine, pour obtenir les dérivés bromés et chlorés correspondants, et que si désiré, l'on traite les produits comportant en position 10 un substituant éthynyl par un halo-succinimide pour obtenir les produits correspondants comportant en position 10 un substituant $-C{\equiv}C-Hal$, et que si désiré l'on acyle ou éthérifie le radical hydroxyle en position 17 béta des produits comportant en position 17 alpha un atome d'hydrogène, un radical $R_4$ ou un radical $-CH_2-CH_2-CH_2OH$.

8. Procédé selon la revendication 7 de préparation des produits de formule $I_{3A}$ telle que définie à la revendication 7, caractérisé en en ce que l'on traite un produit de formule II':

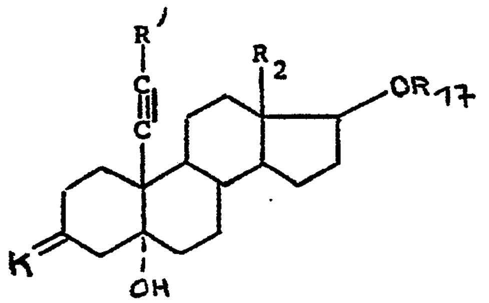

(II')

dans laquelle K, R' et $R_2$ ont la signification indiquée à la revendication 7 et $R_{17}$ représente un atome d'hydrogène ou un groupement protecteur du radical hydroxyle, par un acide et éventuellement par un réactif de déprotection du radical hydroxyle.

9. A titre de médicaments, les produits de formule génerale I telle que définie à la revendication 1.

10. A titre de médicaments, les produits tels que définis à l'une des revendications 2 à 5.

11. A titre de médicaments, les produits de formule générale I dont les noms suivent:
- la gamma-lactone de l'acide 10 béta-éthynyl 17 béta-hydroxy 3-oxo 19-nor 17 alpha-pregn 4-èn 21-carboxylique,
- la 17 béta-hydroxy 10 béta-(1-propynyl) estr-4-èn 3-one,
- la gamma-lactone de l'acide 17 béta-hydroxy 3-oxo 10 béta-(1-propynyl) 19-nor 17 alpha-pregn 4-èn 21-carboxylique.

12. Les compositions pharmaceutiques renfermant, comme principe actif, au moins un médicament défini à l'une quelconque des revendications 9 à 11.

## Claims

1. The products of general formula I:

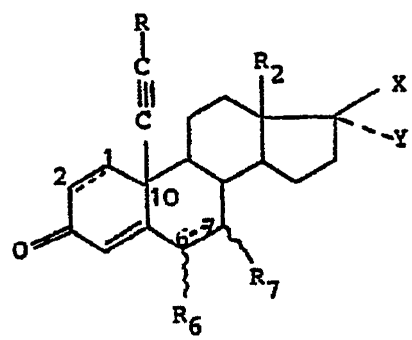

(I)

in which R represents:
- <u>either</u> a hydrogen atom,
- <u>or</u> an alkyl, alkenyl or alkynyl radical having at most 8 carbon atoms possibly substituted by a radical

chosen from the following radicals: hydroxyl, carboxy, methoxycarbonyl, ethoxycarbonyl, amino, tritylamino, chloroacetylamino, trifluoroacetylamino, trichloroethoxycarbonylamino, methylamino, dimethylamino or halogen,

– or an aryl or aralkyl radical optionally substituted by a radical chosen from the following radicals: hydroxyl carboxy, methoxycarbonyl, ethoxycarbonyl, amino, methylamino, dimethylamino, methyl methoxy or methylthio,

– or an alkoxy, carboxy, methoxycarbonyl, ethoxycarbonyl or dialkylamino radical,

– or a hydrogen atom,

– or a trialkylsilyl radical,

$R_6$ and $R_7$ are such that, either $R_6$ and $R_7$ form with the carbons that carry them a cyclopropyl radical, or $R_6$ represents a hydrogen atom and $R_7$ represents the $R_1$ radical, $R_1$ representing:

– either a hydrogen atom,

– or an alkyl, alkenyl or alkynyl radical having at most 6 carbon atoms, optionally substituted by a radical chosen from the following radicals: hydroxyl, carboxy, methoxycarbonyl, ethoxycarbonyl, amino, tritylamino, chloroacetylamino, trifluoroacetylamino, trichloroethoxy-carbonylamino, methylamino, dimethylamino or halogen,

– or an acetylthio radical,

$R_2$ represents a methyl or ethyl radical,

X and Y are such that:

– either X represents a hydroxyl, acetyloxy, propionyloxy, methoxy or ethoxy radical and Y represents either a $-CH_2CH_2CO_2M$ in which M represents a hydrogen atom, an alkali metal atom or an ammonium radical,

– or a $-CH_2CH_2-CH_2OH$ radical

– or X and Y together represent a radical:

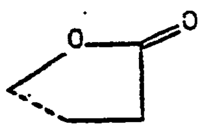

or

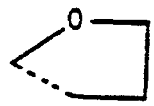

– or X and Y together represent a radical

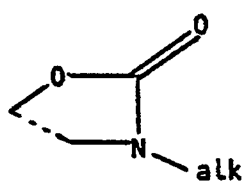

in which alk represents an alkyl radical containing at most 8 carbon atoms,

– or X and Y together represent a radical

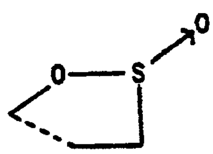

or X represents a hydroxyl radical and Y a

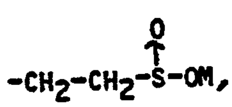

M being defined as above,

– or X represents a hydroxyl, acetyloxy, propionyloxy, methoxy or ethoxy radical and Y represents, either a hydrogen atom or Y represents $R_4$, $R_4$ representing an alkyl, alkenyl or alkynyl radical having at most 6 carbon atoms,

the dotted lines at position 1 (2) and 6 (7) indicate the possible presence of a second bond between the carbons that carry them, it being understood that there cannot be a second bond at position 6 (7) when $R_6$ and $R_7$ form a cyclopropyl radical with the carbons which carry them, the wavy lines at position 6 and 7 indicate that the substituents $R_6$ and $R_7$ can be found at one of the possible positions alpha or beta, it

being understood that R cannot represent a hydrogen atom when $R_6$ and $R_7$ each represent a hydrogen atom, $R_2$ represents a methyl radical, X represents a hydroxy, acetyloxy, propionyloxy, methoxy or ethoxy radical and Y represents a hydrogen atom or $R_4$ and the dotted lines at position 1 (2) and 6 (7) together do not represent a second bond between the carbons which carry them.

2. The products of general formula (I), such as defined in claim 1, corresponding to formula $I_1$:

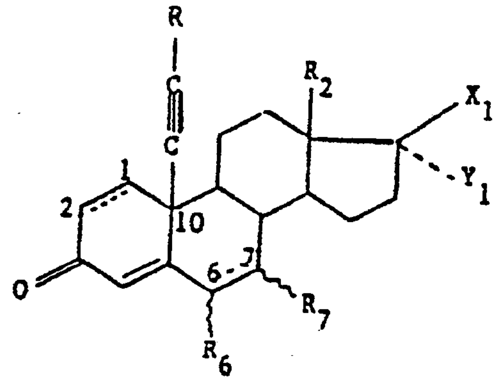
($I_1$)

in which R, $R_6$, $R_7$, $R_2$, the dotted lines at position 1 (2) and 6 (7) and the wavy lines at position 6 and 7 have the meanings indicated in claim 1,
$X_1$ and $Y_1$ are such that:
– either $X_1$ represents a hydroxyl, acetyloxy, propionyloxy, methoxy or ethoxy radical and $Y_1$ represents either a $-CH_2CH_2CO_2M$ radical in which M represents a hydrogen atom, an alkali metal atom or an ammonium radical, or a $-CH_2CH_2-CH_2OH$ radical,
– or $X_1$ and $Y_1$ together represent a radical:

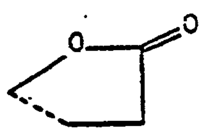
or
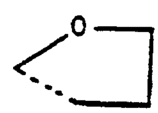

– or $X_1$ represents a hydroxyl, acetyloxy, propionyloxy, methoxy or ethoxy radical and $Y_1$ represents either a hydrogen atom or $Y_1$ represents $R_4$, $R_4$ representing an alkyl, alkenyl or alkynyl radical having at most 6 carbon atoms,
it being understood that R cannot represent a hydrogen atom when $R_6$ and $R_7$ each represent a hydrogen atom, $R_2$ represents a methyl radical, $X_1$ represents a hydroxy, acetyloxy, propionyloxy, methoxy or ethoxy radical, $Y_1$ represents a hydrogen atom or $R_4$, and the dotted lines at position 1 (2) and 6 (7) do not represent a second bond between the carbons which carry them.

3. The products of general formula (I), as defined in claim 1, corresponding to the formula:

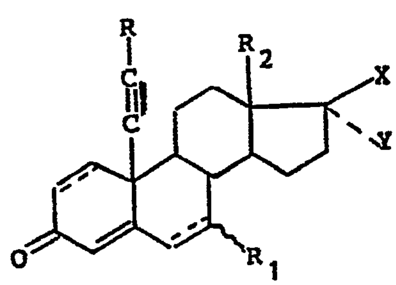

in which R, $R_1$, $R_2$, X, Y, the dotted lines and the wavy lines have the meaning indicated in claim 1.

4. The products of formula I as defined in any one of claims 1 to 3, in which R is chosen from the group formed by the hydrogen atom, the alkyl radicals having from 1 to 3 carbon atoms, the hydroxymethyl radical and the phenyl radical.

5. The products of formula I as defined in any one of the claims 1 to 4, in which the substituent $R_1$ is chosen from the group formed by the hydrogen atom, the alkyl radicals having from 1 to 4 carbon atoms and the acetylthio radical and X and Y are such that either X represents a hydroxy radical and Y represents either a $CH_2CH_2CO_2M'$ radical in which M′ represents a hydrogen atom or an alkali metal atom, or Y represents a hydrogen atom, – or X and Y represent a radical:

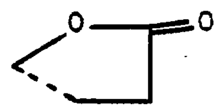

6. The products of general formula (I), which have the following names:
– gamma-lactone of 10-beta-ethynyl-17-beta-hydroxy-3-oxo-19-nor-17-alpha-pregn-4-en-21-carboxylic acid,
– 17-beta-hydroxy-10-beta-(1-propynyl)-estr-4-en-3-one,
– gamma-lactone of 17-beta-hydroxy-3-oxo-10-beta-(1-propynyl)-19-nor-17-alpha-pregn-4-en-21-carboxylic acid.

7. Process for the preparation of the products of formula I, as defined in claim 1, it being understood that R cannot represent a hydrogen atom when $R_6$ and $R_7$ each represent a hydrogen atom, $R_2$ represents a methyl radical, X represents a hydroxy, acetyloxy, propionyloxy, methoxy or ethoxy radical and Y represents a hydrogen atom or $R_4$ and the dotted lines at position 1 (2) and 6 (7) do not together represent as second bond between the carbons which carry them, characterised in that a product of formula II:

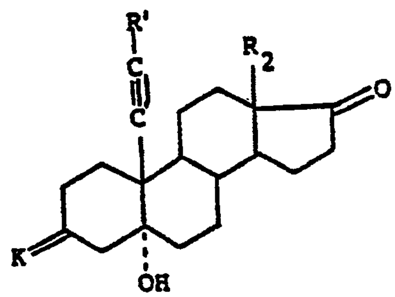

(II)

in which R′ represents either R, R having the meaning indicated in claim 1, or R in which the reactive functions are protected, $R_2$ has the significance indicated in claim 1 and K represents a protector group of the ketone function is treated,

I) <u>either</u> firstly by a trimethylsulphonium halide in the presence of a strong base, then by a metallic derivative of acetonitrile and finally by a base, then by an acid,

– <u>or</u> by a reagent of formula:

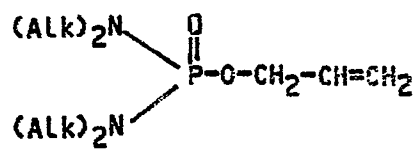

in which Alk represents an alkyl radical having from 1 to 4 carbon atoms in the presence of a strong base then by an acid, to obtain a product of formula $I_A$:

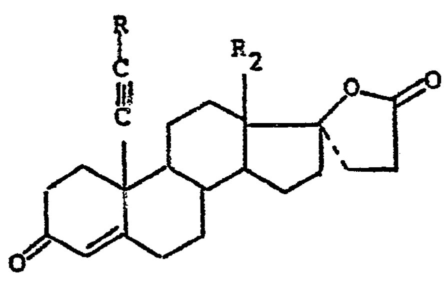

($I_A$)

II) <u>or</u> by a reagent of formula:

$$XMg–CH_2–CH_2–CH_2–OB$$

in which X represents a halogen atom and B represents a protector group of the hydroxyl radical or magnesium alcoholate to obtain, after treatment with an acid a product of formula $I'_A$:

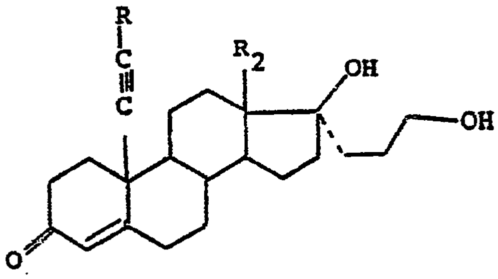

(I'A)

product of formula I'A is optionally treated with a halide of sulphonic acid in the presence of a base to obtain a product of formula I"A:

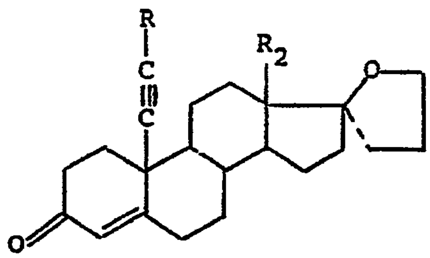

(I"A)

III) <u>or</u> by a reducing agent, then by an acid, to obtain a product of formula $I_{3A}$:

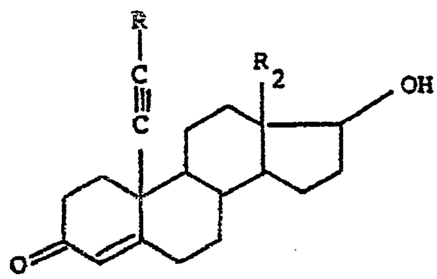

($I_{3A}$)

IV) <u>or</u> by an organometallic compound $R_4MgX$, X representing a halogen atom, then by an acid, to obtain a product of formula $I_{4A}$:

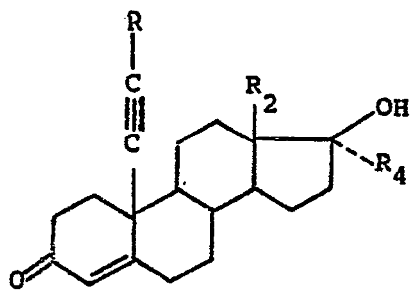

($I_{4A}$)

V) <u>or</u> firstly by trimethylsulphonium halide in the presence of a strong base, then by a primary amine of formula $H_2N$-alk, alk being defined as previously, in the presence of an acid, then by alkyl chloroformate, by a strong base and finally by an acid, to obtain a product of formula $I_{5A}$:

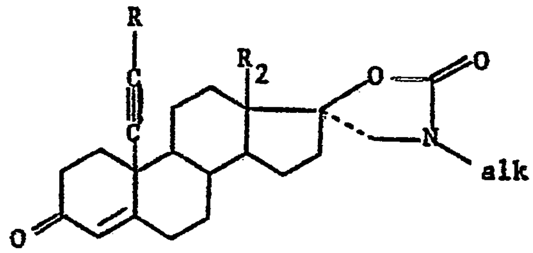

$(I_{5A})$

VI) <u>or</u> firstly by a trimethylsulphonium halide in the presence of a strong base, then by methyl terbutyl sulphoxide in the presence of n-butyl-lithium to obtain a product of formula:

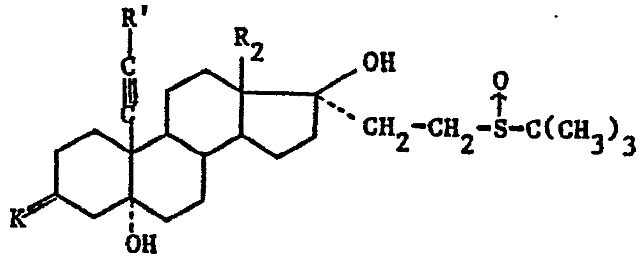

in the form of a mixture of two diastereoisomers at the level of the sulphur atom, if desired the two diastereoisomers are separated, then either their mixture or each of them separately are subjected to the action of an acid, to obtain a product of formula:

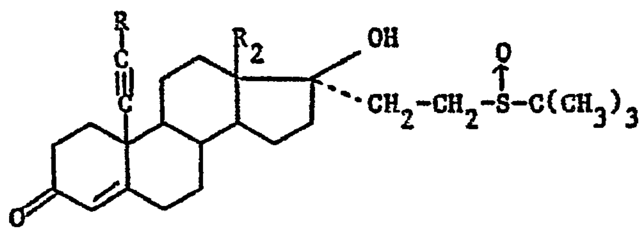

in the form of a mixture of two diastereoisomers or one diastereoisomer, if appropriate, the two diastereoisomers are separated, then either their mixture or each separately are subjected, to the action of N-chloro or N-bromo succinimide, to obtain a product of formula $I_{6A}$:

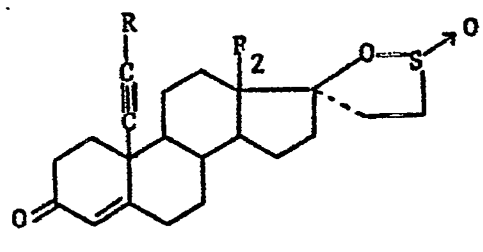

$I_{6A}$

and that, if desired, the products of formulae $I_A$, $I'_A$, $I''_A$, $I_{3A}$, $I_{4A}$, $I_{5A}$ and $I_{6A}$ are treated with an alkyl orthoformate in the presence of an acid, then by a dehydrogenation agent, to obtain the products of the following formulae $I_B$, $I'_B$, $I''_B$, $I_{3B}$, $I_{4B}$, $I_{5B}$ and $I_{6B}$:

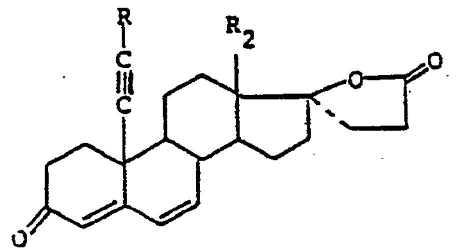

$I_B$

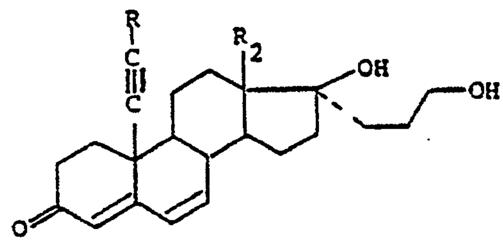

$I'_B$

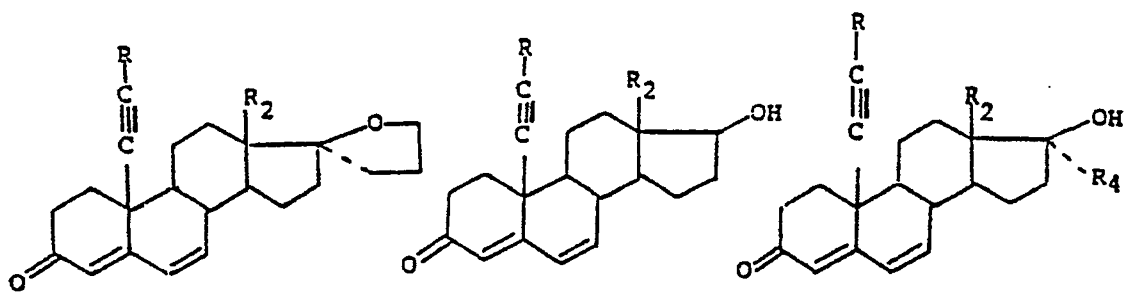

$I''_B$ $I_{3B}$ $I_{4B}$

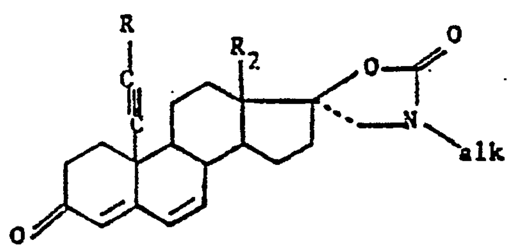

$I_{5B}$

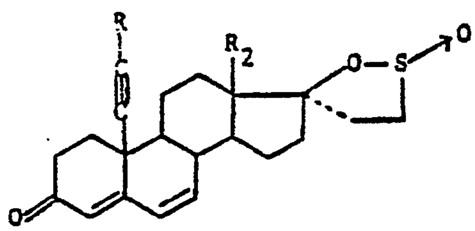

$I_{6B}$

which products of formulae $I_B$; $I'_B$, $I''_B$, $I_{3B}$, $I_{4B}$, $I_{5B}$ and $I_{6B}$ are treated if desired:

– <u>either</u> with an organo magnesium compound of formula $R_1MgX'$ optionally in the presence of a copper salt, formula in which $R_1$ has the significance indicated above and X' represents a halogen atom,

– <u>or</u> by an organo metallic compound of formula $(R_1)_2$ CuLi then by an acid, to obtain the products of the following formulae $I_C$, $I'_C$, $I''_C$, $I_{3C}$, $I_{4C}$, $I_{5C}$ and $I_{6C}$:

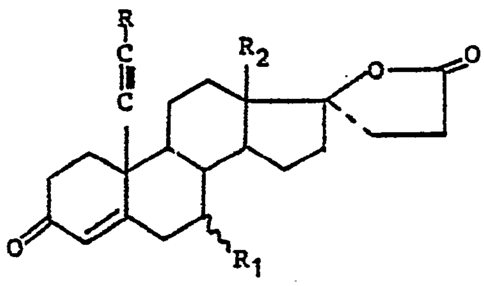

I_C

;

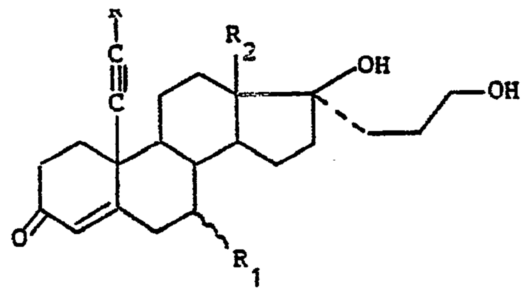

I'_C

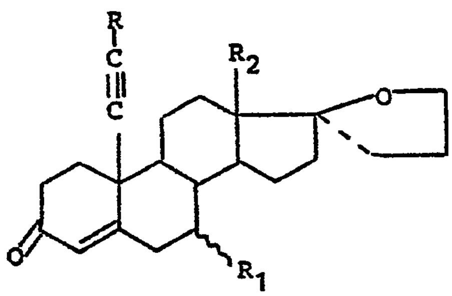

I"_C

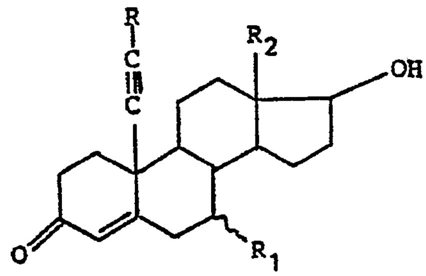

I_3C

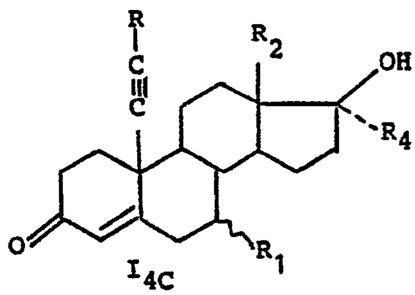

I_4C

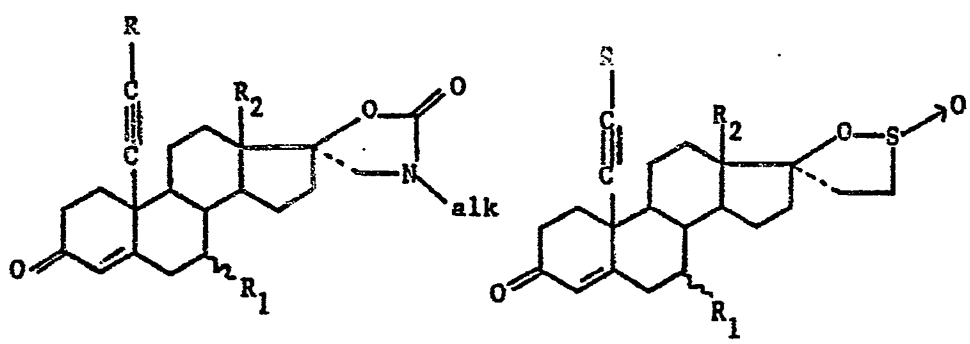

I_5C

I_6C

in the form of a 7 alpha and 7 beta mixture, which mixture, if desired, is separated and if desired the 7 beta products are subjected to a dehydrogenation agent to obtain the corresponding delta 6 (7) products:

– <u>or</u> the products of formulae $I_B$, $I'_B$, $I''_B$, $I_{3B}$, $I_{4B}$, $I_{5B}$ and $I_{6B}$, are subjected to the action of a reagent chosen from the group constituted by trimethylsulphonium iodide and trimethylsulphoxonium iodide in the presence of a strong base to obtain the products of the following formulae $I_D$, $I'_D$, $I''_D$, $I_{3D}$, $I_{4D}$, $I_{5D}$ and $I_{6D}$:

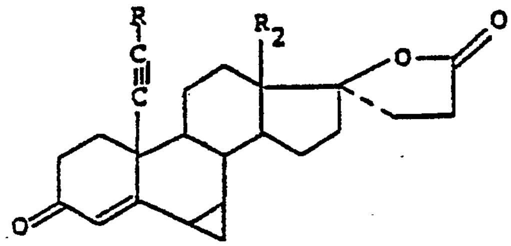

$I_D$

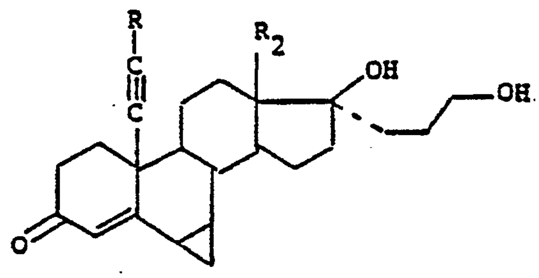

$I'_D$

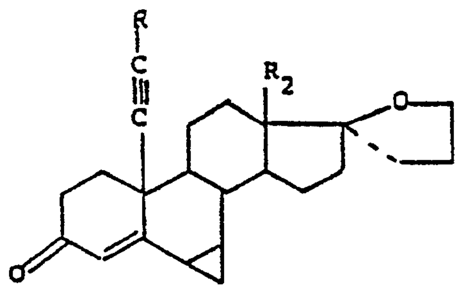

$I''_D$

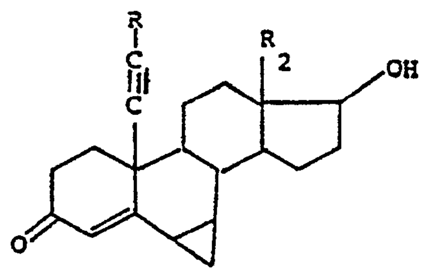

$I_{3D}$

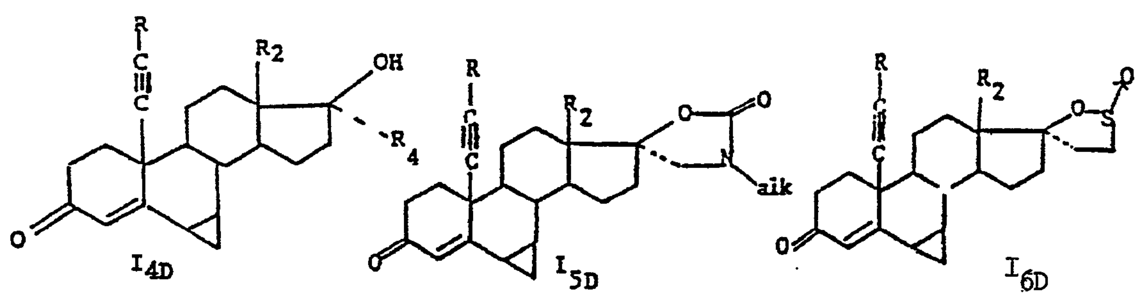

$I_{4D}$ $I_{5D}$ $I_{6D}$

in the form of a mixture of 6 alpha, 7 alpha and 6 beta, 7 beta, and if desired, the isomers thus obtained are separated and that if desired the products $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$ are treated with a dehydrogenation agent or by a micro-organism capable of dehydrogenating the molecule at position 1 (2) to obtain the corresponding products containing an unsaturation at position 1 (2) and that, if desired, the products of formulae $I_A$, $I_B$, $I_C$, $I_D$ and the corresponding products containing an unsaturation at position 1 (2) are treated using an alkaline hydroxide or ammonium hydroxide to obtain the corresponding products in which X represents a hydroxyl radical and Y represents a $-CH_2CH_2CO_2M'$ radical in which M' represents an alkaline metal atom or an ammonium radical and, if desired the products thus obtained are subjected to the action of an acidic agent to obtain the products in which X represents a hydroxyl radical and Y represents a $-CH_2CH_2CO_2H$ radical, and that, if desired the products in which the substituent at position 10 contains a substituent R of a hydroxyalkyl type are treated with a carbon tetrabromide or carbon tetrachloride, in the presence of triphenylphosphine, to obtain the corresponding brominated and chlorinated derivatives, and that, if desired, the products containing at position 10 an ethynyl substituent are treated with a halo-succinimide to obtain the corresponding products containing at position 10 a substituent $-C{\equiv}C-Hal$ and that, if desired the hydroxyl radical at position 17-beta of the products containing at position 17-alpha a hydrogen atom, an $R_4$ radical, or a $-CH_2-CH_2-CH_2OH$ radical, is acylised or etherified.

8. Preparation process according to claim 7, of the products of formula $I_{3A}$, as defined in claim 7, characterised in that a product of formula II':

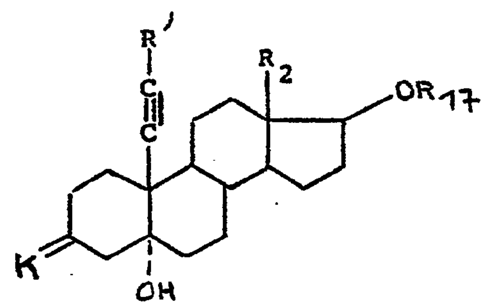

(II')

in which K, R' and $R_2$ have the meaning indicated in claim 7 and $R_{17}$ represents a hydrogen atom or a protector group of a hydroxyl radical is treated, by an acid and optionally by a deprotection reagent of the hydroxyl radical.

9. As medicaments, the products of general formula I as defined in claim 1.

10. As medicaments, the products as defined in one of claims 2 to 5.

11. As medicaments, the products of general formula I the names of which follow:

- gamma-lactone of 10-beta-ethynyl-17-beta-hydroxy-3-oxo-19-nor-17-alpha-pregn-4-en-21-carboxylic acid,
- 17-beta-hydroxy-10-beta-(1-propynyl)-estr-4-en-one,
- gamma-lactone of 17-beta-hydroxy-3-oxo-10-beta-(1-propynyl)-19-nor-17-alpha-pregn-4-en-21-carboxylic acid.

12. The pharmaceutical compositions containing, as active principle, at least one medicament defined in any of claims 9 to 11.

**Patentansprüche**

1. Produkte der allgemeinen Formel I

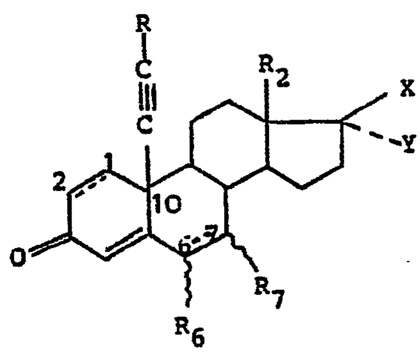

(I)

worin R bedeutet:
- entweder ein Wasserstoffatom
- oder einen Alkyl-, Alkenyl- oder Alkinylrest mit höchstens 8 Kohlenstoffatomen, der gegebenenfalls durch einen Rest, ausgewählt unter den Hydroxyl-, Carboxy-, Methoxycarbonyl-, Ethoxycarbonyl-, Amino-, Tritylamino-, Chloracetylamino-,Trifluoracetylamino-, Trichlorethoxycarbonylamino-, Methylamino-, Dimethylamino- oder Halogenresten, substituiert ist,
- oder einen Aryl- oder Aralkylrest, der gegebenenfalls durch einen Rest, ausgewählt unter den Hydroxyl-, Carboxy-, Methoxycarbonyl-, Ethoxycarbonyl-, Amino-, Methylamino-, Dimethylamino-, Methylmethoxy- oder Methylthioresten, substituiert ist,
- oder einen Alkoxy-, Carboxy-, Methoxycarbonyl-, Ethoxycarbonyl- oder Dialkylaminorest,
- oder ein Halogenatom,
- oder einen Trialkylsilylrest,

$R_6$ und $R_7$ derart sind, daß entweder $R_6$ und $R_7$ mit den Kohlenstoffatomen, die sie tragen, einen Cyclopropylrest bilden oder $R_6$ ein Wasserstoffatom bedeutet und $R_7$ für einen Rest $R_1$ steht, wobei $R_1$ bedeutet:
- entweder ein Wasserstoffatom
- oder einen Alkyl-, Alkenyl- oder Alkinylrest mit höchstens 6 Kohlenstoffatomen, der gegebenenfalls substituiert ist durch einen Rest, ausgewählt unter den Hydroxy-, Carboxy-, Methoxycarbonyl-, Ethoxycarbonyl-, Amino-, Tritylamino-, Chloracetylamino-, Trifluoracetylamino-,Trichlorethoxycarbonylamino-, Methylamino-, Dimethylamino- oder Halogenresten,
- oder einen Acetylthiorest,

$R_2$ einen Methyl- oder Ethylrest bedeutet,
X und Y derart sind, daß
– entweder X einen Hydroxyl-, Acetyloxy-, Propionyloxy-, Methoxy- oder Ethoxyrest bedeutet und Y entweder einen Rest $CH_2CH_2CO_2M$, worin M für ein Wasserstoffatom, ein Alkalimetallatom oder eine Ammoniumgruppe steht,
oder einen Rest $-CH_2CH_2-CH_2OH$ wiedergibt,
– oder X und Y gemeinsam einen Rest

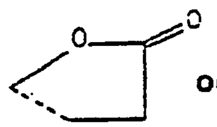
oder
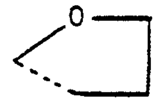

bilden,
– oder X und Y gemeinsam einen Rest

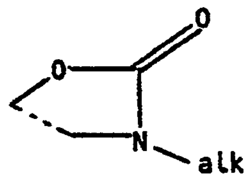

bilden, worin alk für einen Alkylrest mit höchstens 8 Kohlenstoffatomen steht,
– oder X und Y gemeinsam einen Rest

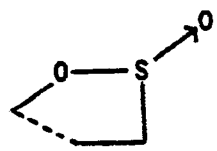

bilden
oder X einen Hydroxylrest bedeutet und Y einen Rest

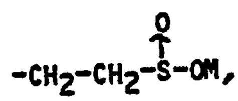

wiedergibt, wobei M wie vorstehend definiert ist,
– oder X einen Hydroxyl-, Acetyloxy-, Propionyloxy-, Methoxy- oder Ethoxyrest bedeutet und Y entweder ein Wasserstoffatom wiedergibt oder Y die Bedeutung von $R_4$ besitzt, wobei $R_4$ einen Alkyl-, Alkenyl- oder Alkinylrest mit höchstens 6 Kohlenstoffatomen bedeutet,
die gestrichelten Linien in 1(2)- und 6(7)-Stellung die etwaige Anwesenheit einer zweiten Bindung zwischen den Kohlenstoffatomen, die sie enthalten, anzeigen, wobei es sich versteht, daß keine zweite Bindung in 6(7)-Stellung vorliegen kann, wenn $R_6$ und $R_7$ mit den Kohlenstoffatomen, die sie tragen, einen Cyclopropylrest bilden,
die gewellten Linien in 6- und 7-Stellung anzeigen, daß die Substituenten$R_6$ und $R_7$ sich in einer der möglichen α- oder β-Positionen befinden können, wobei es sich versteht, daß R kein Wasserstoffatom wiedergeben kann, wenn $R_6$ und $R_7$ jeweils ein Wasserstoffatom bedeuten, $R_2$ einen Methylrest darstellt, X einen Hydroxy-, Acetyloxy-, Propionyloxy-, Methoxy- oder Ethoxyrest wiedergibt und Y für ein Wasserstoffatom steht, oder $R_4$ und die gestrichelten Linien in 1(2)- und 6(7)-Stellung nicht gemeinsam eine zweite Bindung zwischen den Kohlenstoffatomen, die sie tragen, wiedergeben.

2. Produkte der allgemeinen Formel (I) gemäß Anspruch 1 der Formel $I_1$

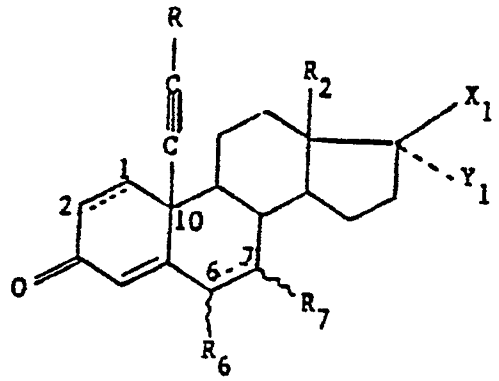

$(I_1)$

worin R, $R_6$, $R_7$, $R_2$, die gestrichelten Linien in 1(2)- und 6(7)-Stellung und die gewellten Linien in 6- und 7-Stellung die in Anspruch 1 angegebenen Bedeutungen besitzen,
$X_1$ und $Y_1$ derart sind, daß
– entweder $X_1$ für einen Hydroxyl-, Acetyloxy-, Propionyloxy-, Methoxy- oder Ethoxyrest steht und $Y_1$ entweder einen Rest $-CH_2CH_2CO_2M$, worin M für ein Wasserstoffatom, ein Alkalimetallatom oder eine Ammoniumgruppe steht, oder einen Rest $-CH_2CH_2-CH_2OH$ wiedergibt,
– oder $X_1$ und $Y_1$ gemeinsam einen Rest

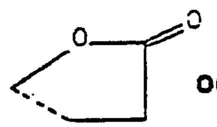

oder

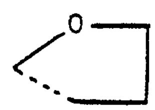

bilden,
– oder $X_1$ einen Hydroxyl-, Acetyloxy-, Propionyloxy-, Methoxy- oder Ethoxyrest bedeutet und $Y_1$ entweder ein Wasserstoffatom wiedergibt oder $Y_1$ die Bedeutung von $R_4$ besitzt, wobei $R_4$ einen Alkyl-, Alkenyl- oder Alkinylrest mit höchstens 6 Kohlenstoffatomen darstellt, wobei es sich versteht, daß R kein Wasserstoffatom bedeuten kann, wenn $R_6$ und $R_7$ jeweils ein Wasserstoffatom bedeuten, $R_2$ einen Methylrest wiedergibt, $X_1$ für eine Hydroxy-, Acetyloxy-, Propionyloxy-, Methoxy- oder Ethoxygruppe steht, $Y_1$ ein Wasserstoffatom oder $R_4$ bedeutet und die gestrichelten Linien in 1(2)- und 6(7)-Stellung keine zweite Bindung zwischen den Kohlenstoffatomen, die sie aufweisen, darstellen.

3. Produkte der allgemeinen Formel (I) gemäß Anspruch 1 der Formel

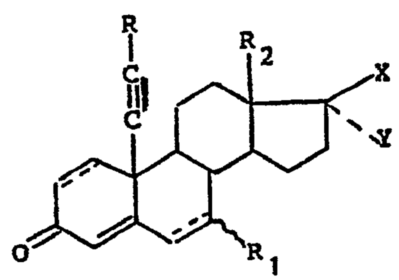

worin R, $R_1$, $R_2$, X, Y, die gestrichelten Linien und die gewellten Linien die in Anspruch 1 angegebene Bedeutung besitzen.

4. Produkte der Formel I gemäß einem der Ansprüche 1 bis 3, worin R ausgewählt ist unter Wasserstoff, den Alkylresten mit 1 bis 3 Kohlenstoffatomen, der Hydroxymethylgruppe und dem Phenylrest.

5. Produkte der Formel I gemäß einem der Ansprüche 1 bis 4, worin der Substituent $R_1$ ausgewählt ist unter Wasserstoff, den Alkylresten mit 1 bis 4 Kohlenstoffatomen und dem Acethylthiorest und X und Y derart sind, daß entweder X für eine Hydroxygruppe steht und Y entweder eine Gruppe $CH_2CH_2CO_2M'$, worin M' für ein Wasserstoffatom oder ein Alkalimetallatom steht, wiedergibt oder Y ein Wasserstoffatom bedeutet,
– oder X und Y für einen Rest

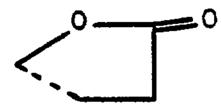

stehen.

6. Die Produkte der allgemeinen Formel (I) mit den folgenden Bezeichnungen:
γ-Lacton der 10β–Ethinyl-17β-hydroxy-3-oxo-19-nor-17α-pregn-4-en-21-carbonsäure,
17β-Hydroxy-10β-(1-propinyl)-östr-4-in-3-on,
γ-Lacton der 17β-Hydroxy–3-oxo-10β-(1-propinyl)-19-nor-17α-pregn-4-en-21-carbonsäure.

7. Verfahren zur Herstellung der Produkte der Formel I gemäß Anspruch 1, wobei es sich versteht, daß R kein Wasserstoffatom bedeuten kann, wenn $R_6$ und $R_7$ jeweils ein Wasserstoffatom wiedergeben, $R_2$ einen Methylrest bedeutet, X für eine Hydroxy-, Acetyloxy-, Propionyloxy-, Methoxy- oder Ethoxygruppe steht und Y ein Wasserstoffatom oder $R_4$ bedeutet und die gestrichelten Linien in 1(2)- und 6(7)-Stellung nicht gemeinsam eine zweite Bindung zwischen den Kohlenstoffatomen, die sie aufweisen, bilden, dadurch gekennzeichnet, daß man ein Produkt der Formel II

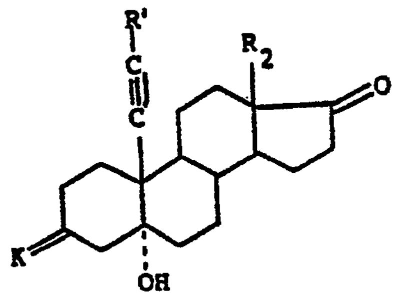

(II)

worin R′ entweder die Bedeutung von R besitzt, wobei R die in Anspruch 1 angegebene Bedeutung besitzt, oder die Bedeutung von R aufweist, worin die reaktiven Funktionen geschützt sind, $R_2$ die in Anspruch 1 angegebene Bedeutung besitzt und K für eine Schutzgruppe der Ketonfunktion steht,
(I) <u>entweder</u> zunächst mit einem Trimethylsulfoniumhalogenid in Gegenwart einer starken Base, dann mit einem Metallderivat des Acetonitrils und schließlich mit einer Base, danach mit einer Säure behandelt, <u>oder</u> zunächst mit einem Reagens der Formel

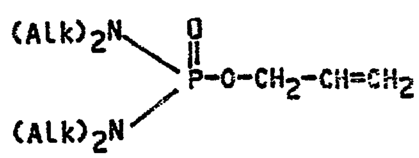

worin Alk für einen Alkylrest mit 1 bis 4 Kohlenstoffatomen steht, in Gegenwart einer starken Base, dann mit einer Säure behandelt, um zu einem Produkt der Formel $I_A$

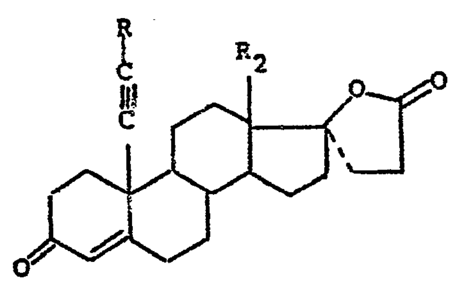

$(I_A)$

zu gelangen;
(II) <u>oder</u> mit einem Reagens der Formel

$$XMg–CH_2–CH_2–CH_2–OB$$

worin X für ein Halogenatom steht und B eine Schutzgruppe für den Hydroxylrest wiedergibt, oder einem Magnesiumalkoholat behandelt, um nach Behandlung mit einer Säure zu einem Produkt der Formel $I'_A$

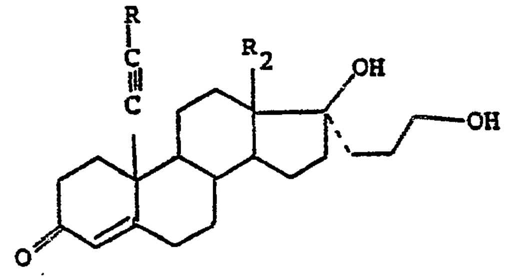

($I'_A$)

zu gelangen, das Produkt der Formel $I'_A$ gegebenenfalls mit einem Sulfonsäurehalogenid in Anwesenheit einer Base behandelt, um zu einem Produkt der Formel $I''_A$

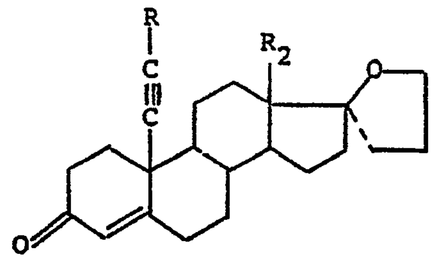

($I''_A$)

zu gelangen;

(III) oder mit einem Reduktionsmittel, dann mit einer Säure behandelt, um zu einem Produkt der Formel $I_{3A}$

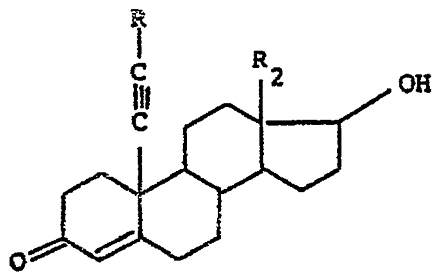

($I_{3A}$)

zu gelangen;

(IV) oder mit einer organometallischen Verbindung $R_4MgX$, worin X für ein Halogenatom steht, dann mit einer Säure behandelt, um zu einem Produkt der Formel $I_{4A}$

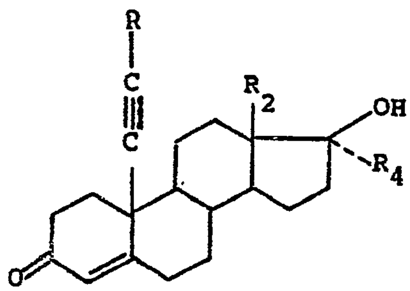

($I_{4A}$)

zu gelangen;

(V) oder zunächst mit einem Trimethylsulfoniumhalogenid in Gegenwart einer starken Base, dann mit einem primären Amin der Formel $H_2N$-alk, worin alk wie vorstehend definiert ist, in Anwesenheit einer Säure, dann mit einem Alkylchlorformiat, mit einer starken Base und schließlich mit einer Säure behandelt, um zu einem Produkt der Formel $I_{5A}$

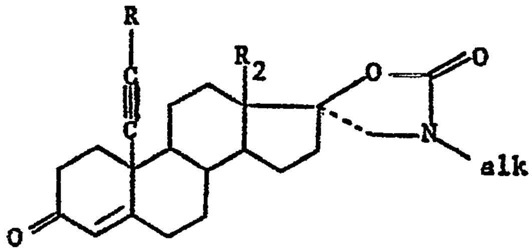

($I_{5A}$)

zu gelangen;
(VI) oder zunächst mit einem Trimethylsulfoniumhalogenid in Anwesenheit einer starken Base, dann mit Methyl-tert.-butylsulfoxid in Gegenwart von n-Butyllithium behandelt, um zu einem Produkt der Formel

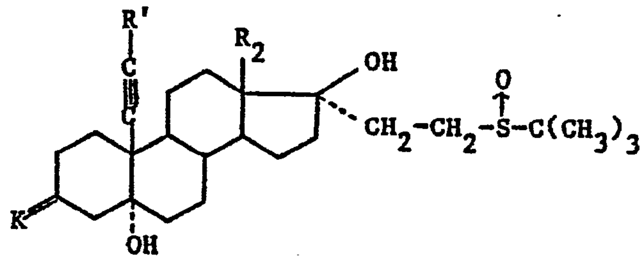

in Form eines Gemisches der beiden Diastereomeren hinsichtlich des Schwefelatoms zu gelangen, gewünschtenfalls die beiden Diastereomeren trennt, hiernach deren Gemisch oder ein jedes von ihnen getrennt der Einwirkung einer Säure unterzieht, um zu einem Produkt der Formel

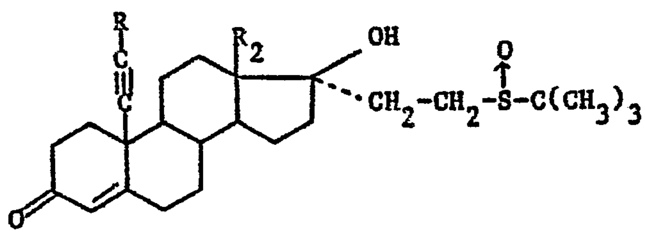

in Form eines Gemisches der beiden Diastereomeren oder eines Diastereomeren zu gelangen, gegebenenfalls die Diastereomeren trennt, hiernach entweder deren Gemisch oder ein jedes von ihnen getrennt der Einwirkung von N-Chlor- oder N-Bromsuccinimid unterzieht, um zu einem Produkt der Formel $I_{6A}$

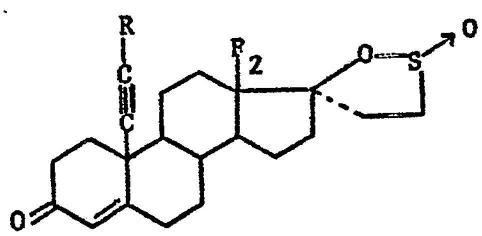

$I_{6A}$

zu gelangen, und gewünschtenfalls die Produkte der Formeln $I_A$, $I'_A$, $I''_A$, $I_{3A}$, $I_{4A}$, $I_{5A}$, und $I_{6A}$ mit einem Alkylorthoformiat in Gegenwart einer Säure, dann mit einem Dehydrierungsmittel behandelt, um zu den folgenden Produkten der Formeln $I_B$, $I'_B$, $I''_B$, $I_{3B}$, $I_{4B}$, $I_{5B}$, und $I_{6B}$

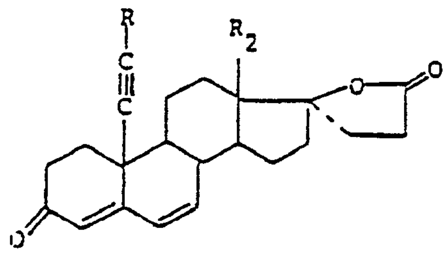

$I_B$

;

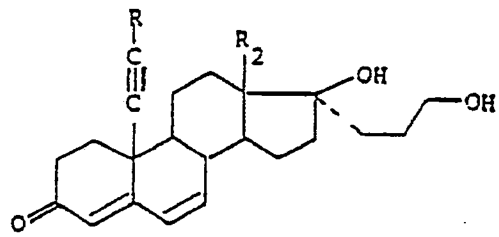

$I'_B$

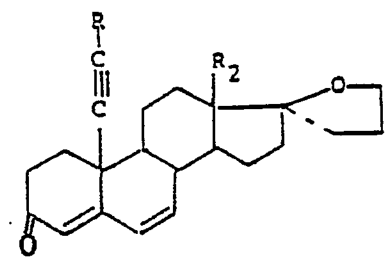

$I''_B$

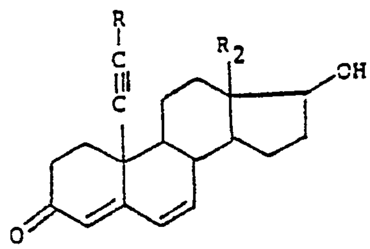

$I_{3B}$

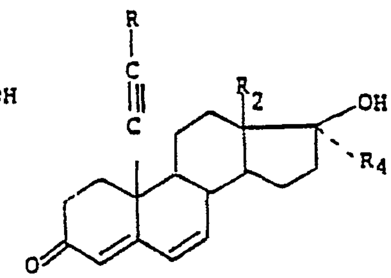

$I_{4B}$

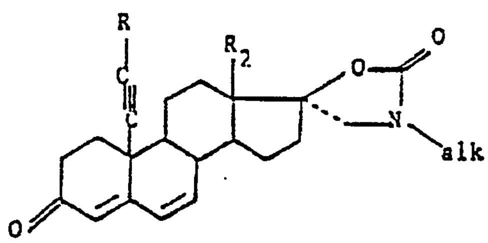

$I_{5B}$

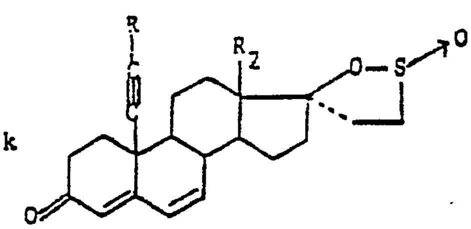

$I_{6B}$

zu gelangen, die Produkte der Formeln $I_B$, $I'_B$, $I''_B$, $I_{3B}$, $I_{4B}$, $I_{5B}$, und $I_{6B}$ man gewünschtenfalls

– entweder mit einer Organomagnesiumverbindung der Formel $R_1MgX'$, worin $R_1$ die vorstehend angegebene Bedeutung besitzt und X' ein Halogenatom bedeutet, gegebenenfalls in Anwesenheit eines Kupfersalzes behandelt

– oder mit einer organometallischen Verbindung der Formel $(R_1)_2CuLi$, dann mit einer Säure behandelt, um zu den Produkten der folgenden Formeln $I_C$, $I'_C$, $I''_C$, $I_{3C}$, $I_{4C}$, $I_{5C}$, und $I_{6C}$

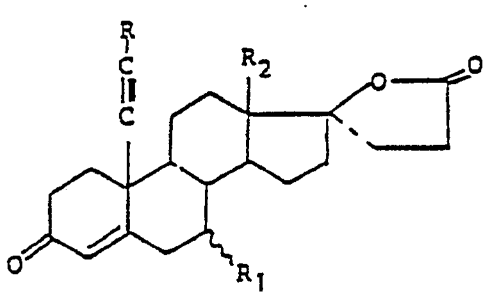

$I_C$

;

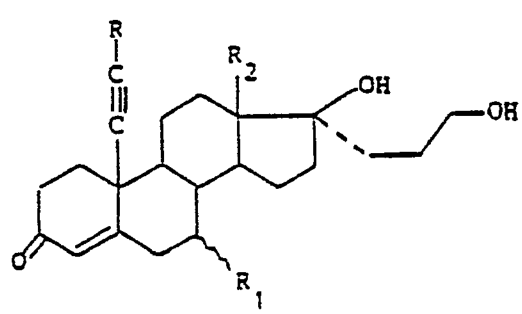

$I'_C$

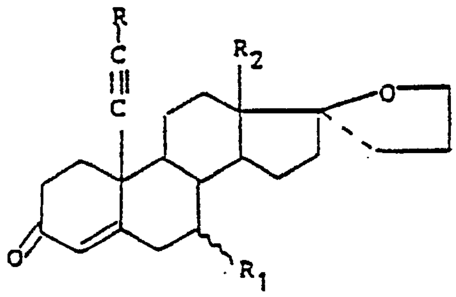

I"C

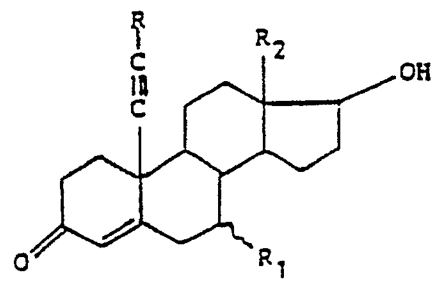

I3C

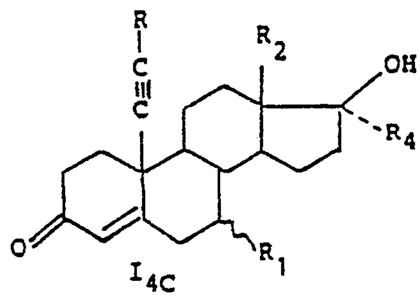

I4C

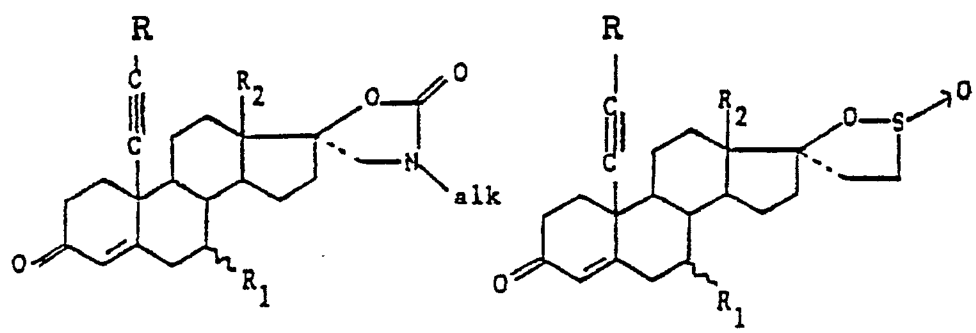

in Form eines 7α-7β-Gemisches zu gelangen, das Gemisch gegebenenfalls trennt und gewünschtenfalls die 7β-Produkte einem Dehydrierungsreagens unterzieht, um zu den entsprechenden Δ-6(7)-Produkten zu gelangen,

– oder die Produkte der Formeln $I_B$, $I'_B$, $I''_B$, $I_{3B}$, $I_{4B}$, $I_{5B}$, und $I_{6B}$ der Einwirkung eines Reagens, ausgewählt unter Trimethylsulfoniumjodid und Trimethylsulfoxoniumjodid, in Anwesenheit einer starken Base unterzieht, um zu den Produkten der folgenden Formeln $I_D$; $I'_D$, $I''_D$, $I_{3D}$, $I_{4D}$, $I_{5D}$ und $I_{6D}$

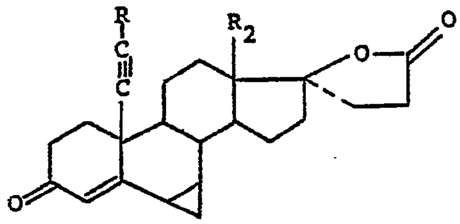

$I_D$

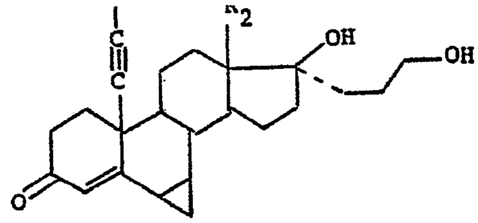

$I'_D$

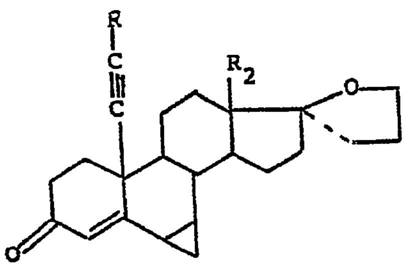

$I''_D$

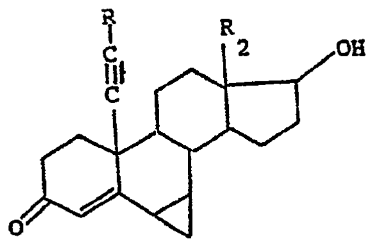

$I_{3D}$

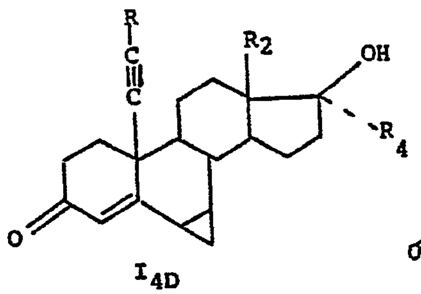

$I_{4D}$

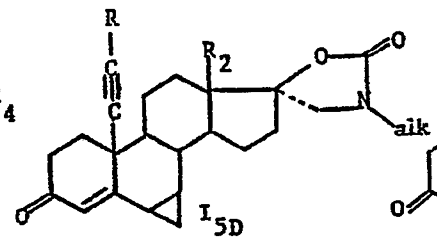

$I_{5D}$

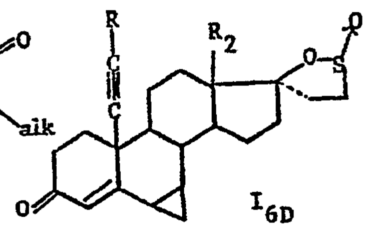

$I_{6D}$

in Form eines 6α-, 7α- und 6β-, 7β-Gemisches zu gelangen, und gewünschtenfalls die so erhaltenen Isomeren trennt und gewünschtenfalls die Produkte $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$ mit einem Dehydrierungsreagens oder mit einem Mikroorganismus, der zur Dehydrierung des Moleküls in 1(2)-Stellung befähigt ist, behandelt, um zu den entsprechenden Produkten mit einer Unsättigung in 1(2)-Stellung zu gelangen, und gewünschtenfalls die Produkte der Formeln $I_A$, $I_B$, $I_C$ und $I_D$ und die entsprechenden Produkte mit einer Unsättigung in 1(2)-Stellung mit Hilfe eines Alkalihydroxids oder mit Hilfe von Ammoniak behandelt, um zu den entsprechenden Produkten zu gelangen, worin X für eine Hydroxylgruppe steht und Y einen Rest $-CH_2CH_2CO_2M'$ wiedergibt, in dem M' ein Alkalimetallatom oder eine Ammoniumgruppe bedeutet, und gewünschtenfalls die so erhaltenen Produkte der Einwirkung eines sauren Mittels unterzieht, um zu den Produkten zu gelangen, worin X für einen Hydroxylrest steht und Y einen Rest $-CH_2CH_2CO_2H$ bedeutet, und gewünschtenfalls die Produkte, worin der Substituent in 10-Stellung einen Substituenten R vom Hydroxyalkyl-Typ aufweist, mit Tetrabrom- oder Tetrachlorkohlenstoff in Gegenwart von Triphenylphosphin behandelt, um zu den entsprechenden bromierten und chlorierten Derivaten zu gelangen, und gewünschtenfalls die Produkte mit einem Ethinylsubstituenten in 10-Stellung mit einem Halosuccinimid behandelt, um zu den entsprechenden Produkten mit einem Substituenten $-C{\equiv}C\text{-Hal}$ in 10-Stellung zu gelangen, und gewünschtenfalls den Hydroxylrest in 17β-Stellung der Produkte, die in 17α-Stellung ein Wasserstoffatom, einen Rest R4 oder einen Rest $-CH_2-CH_2-CH_2OH$ aufweisen, acyliert oder verethert.

8. Verfahren gemäß Anspruch 7 zur Herstellung der Produkte der Formel $I_{3A}$ gemäß Anspruch 7, dadurch gekennzeichnet, daß man ein Produkt der Formel II'

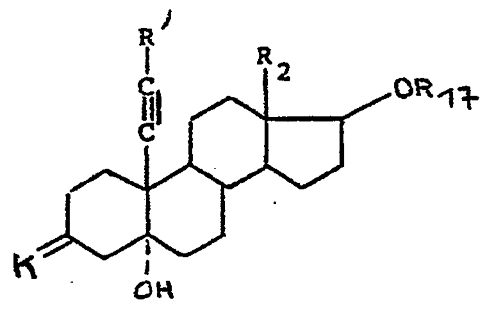

(II')

worin K, R' und $R_2$ wie in Anspruch 7 definiert sind und $R_{17}$ ein Wasserstoffatom oder eine Schutzgruppe für die Hydroxylgruppe wiedergibt, mit einer Säure und gegebenenfalls mit einem Reagens zur Abspaltung der Schutzgruppe des Hydroxylrestes behandelt.

9. Als Arzneimittel die Produkte der allgemeinen Formel I gemäß Anspruch 1.

10. Als Arzneimittel die Produkte gemäß einem der Ansprüche 2 bis 5.

11. Als Arzneimittel die Produkte der allgemeinen Formel I mit den folgenden Bezeichnungen:
γ-Lacton der 10β-Ethinyl-17β-hydroxy-3-oxo-19-nor-17α-pregn-4-en-21-carbonsäure,
17β-Hydroxy-10β-(1-propinyl)-östr-4-en-3-on,
γ-Lacton der 17β-Hydroxy-3-oxo-10β-(1-propinyl)-19-nor-17α-pregn-4-en-21-carbonsäure.

12. Pharmazeutische Zusammensetzungen, die als Wirkstoff zumindest ein Arzneimittel gemäß einem der Ansprüche 9 bis 11 enthalten.